# EUROPEAN PATENT APPLICATION

(11) **EP 4 721 762 A2**
(43) Date of publication of application: **08.04.2026**
(21) Application number: 25217262.2
(22) Date of filing: 23.06.2020
(51) Int. Cl.: A61K 47/34

(54) **MODIFIED POLYAMINE POLYMERS FOR DELIVERY OF BIOMOLECULES INTO CELLS**

(30) Priority: 24.06.2019 US 201962865638 P
(62) Divisional of application: 20740133.2
(71) Applicant: Promega Corporation, Madison, WI 53711-5399 (US)
(72) Inventor: ZHOU, Wenhui, Madison, 53711-5399 (US); HOANG, Trish, Madison, 53711-5399 (US); EGGERS, Christopher Todd, Madison, 53711-5399 (US); WANG, Hui, Madison, 53711-5399 (US); MACHLEIDT, Thomas, Madison, 53711-5399 (US); BINKOWSKI, Brock, Madison, 53711-5399 (US); MEISENHEIMER, Poncho, Madison, 53711-5399 (US); FAN, Frank, Madison, 53711-5399 (US); WOOD, Keith, Madison, 53711-5399 (US)
(74) Representative: Potter Clarkson

(57) **Abstract**

Provided herein are compounds, compositions, and methods for delivering biomolecules to cells. In particular, the present disclosure provides modified polyamine polymers, including polyamine polymers having fluorinated substituents.

## Description

### CROSS-REFERENCE TO RELATED APPLICATION

This application claims priority to U.S. Provisional Patent Application No. 62/865,638, filed on June 24, 2019, the entire contents of which are fully incorporated herein by reference.

### INCORPORATION-BY-REFERENCE OF MATERIAL SUBMITTED ELECTRONICALLY

Incorporated by reference in its entirety herein is a computer-readable nucleotide/amino acid sequence listing submitted concurrently herewith and identified as follows: One 10,000 Byte ASCII (Text) file named "36754-601_ST25," created on June 23, 2020.

### FIELD

Provided herein are compounds, compositions, and methods for delivering biomolecules to cells. In particular, the present disclosure provides modified polyamine polymers, including polyamine polymers having fluorinated substituents.

### BACKGROUND

Cationic polymers, such as polyethyleneimines (PEIs) and poly(amidoamine) (PAMAM) dendrimers, are widely used as carriers to introduce exogenous genes into cells. These materials are easy to manufacture and have superior safety compared with viral gene delivery. However, their commercial and clinical applications are limited by relatively low transfection efficacy and poor cell viability.

### SUMMARY

Provided herein are compounds, compositions, and methods for delivering biomolecules to cells. In particular, the present disclosure provides modified polyamine polymers, including polyamine polymers, having fluorinated substituents.

Embodiments of the present disclosure include a compound or a salt thereof, the compound comprising:
a polyethyleneimine polymer; and
a plurality of substituents bound to amino groups of the polyethyleneimine polymer,
wherein each substituent independently has a formula (I):

-X-(CH₂)ₙ-Z (I),

wherein:
X is a bond or -C(O)-O-;
n is 0, 1, 2, 3, 4, or 5; and
Z is selected from a haloalkyl group, an aryl group, a substituted aryl group, a heteroaryl group, and a substituted heteroaryl group.

In some embodiments, the polyethyleneimine polymer has a weight average molecular weight of about 500 Da to about 250000 Da. In some embodiments, the polyethyleneimine polymer has a weight average molecular weight of about 500 Da to about 2000 Da. In some embodiments, the polyethyleneimine polymer has a weight average molecular weight of about 5000 Da to about 25000 Da.

In some embodiments, the polyethyleneimine polymer is a branched polyethyleneimine polymer. In some embodiments, the polyethyleneimine polymer is a linear polyethyleneimine polymer.

In some embodiments, Z is a haloalkyl group having the following formula: -(CF₂)ₘ-CF₃, wherein m is 1, 2, 3, 4, 5, 6, 7, 8, 9, or 10. In some embodiments, Z is a pentafluorophenyl group. In some embodiments, Z is an unsubstituted pyridyl group.

In some embodiments, X is -C(O)O-, and n is 1 or 2.

In some embodiments, Z is a haloalkyl group having the following formula: -(CF₂)ₘ-CF₃, wherein m is 1, 2, 3, 4, or 5.

In some embodiments, X is a bond, and n is 1 or 2.

In some embodiments, about 0.1 mol% to about 60 mol% of the amino groups of the polyethyleneimine polymer are bound to a substituent of formula (I). In some embodiments, about 5 mol% to about 50 mol% of the amino groups of the polyethyleneimine polymer are bound to a substituent of formula (I). In some embodiments, about 8 mol% to about 40 mol% of the amino groups of the polyethyleneimine polymer are bound to a substituent of formula (I).

Embodiments of the present disclosure also include a compound or a salt thereof, the compound comprising:
a poly(amidoamine) dendrimer; and
a plurality of substituents bound to amino groups of the poly(amidoamine) dendrimer, wherein each substituent independently has a formula (I):

   -X-(CH₂)ₙ-Z (I),
wherein:
   X is a bond or -C(O)-O-;
   n is 0, 1 or 2; and
   Z is selected from a haloalkyl group, an aryl group, a substituted aryl, a heteroaryl group, and a substituted heteroaryl group.

In some embodiments, the poly(amidoamine) dendrimer is a Generation 1, Generation 2, Generation 3, Generation 4, Generation 5, Generation 6, Generation 7, Generation 8, Generation 9, or Generation 10 poly(amidoamine) dendrimer. In some embodiments, the poly(amidoamine) dendrimer is a Generation 1, Generation 2, Generation 3, or Generation 4 poly(amidoamine) dendrimer.

In some embodiments, Z is a haloalkyl group having the following formula: -(CF₂)ₘ-CF₃, wherein m is 1, 2, 3, 4, 5, 6, 7, 8, 9, or 10. In some embodiments, Z is a pentafluorophenyl group. In some embodiments, Z is an unsubstituted pyridyl group.

In some embodiments, X is -C(O)O-. In some embodiments, Z is a haloalkyl group having the following formula: -(CF₂)ₘ-CF₃, wherein m is 1, 2, 3, 4, or 5.

In some embodiments, X is a bond, and n is 1.

In some embodiments, about 0.1 mol% to about 80 mol% of the primary amino groups of the poly(amidoamine) dendrimer are bound to a substituent of formula (I). In some embodiments, about 10 mol% to about 70 mol% of the primary amino groups of the poly(amidoamine) dendrimer are bound to a substituent of formula (I). In some embodiments, about 20 mol% to about 70 mol% of the primary amino groups of the poly(amidoamine) dendrimer are bound to a substituent of formula (I).

Embodiments of the present disclosure also include a method of delivering a biomolecule to a cell, comprising: contacting the cell with an effective amount of a compound described herein (i.e. a compound comprising a polyethylene imine polymer and a plurality of substituents of formula (I), or a compound comprising a poly(amidoamine) dendrimer and a plurality of substituents of formula (I)), or a salt thereof; and contacting the cell with the biomolecule. In some embodiments, the method comprises contacting the cells with an effective amount of two or more different compounds or salts thereof.

In some embodiments, the biomolecule is at least one of a deoxyribonucleic acid (DNA) molecule, a ribonucleic acid (RNA) molecule, a peptide, a polypeptide, a protein, or any combinations or derivatives thereof. In some embodiments, the biomolecule is a deoxyribonucleic acid (DNA) molecule or a ribonucleic acid (RNA) molecule. In some embodiments, the biomolecule is a peptide or polypeptide capable of luminescent activity. In some embodiments, the biomolecule comprises a polypeptide sequence of SEQ ID NO: 4 (LgBiT). In some embodiments, the biomolecule is a ribonucleoprotein complex comprising a Cas9 protein. In some embodiments, the ribonucleoprotein complex further comprises a guide RNA (gRNA) and a donor DNA template, wherein the donor DNA template comprises a sequence encoding a polypeptide from SEQ ID NO: 3.

In some embodiments, the method comprises mixing the compound and the biomolecule to form a mixture and subsequently contacting the cell with the mixture.

Embodiments of the present disclosure also include a kit comprising a compound or a salt thereof, wherein the compound, or salt thereof, is a compound described herein (i.e. a compound comprising a polyethylene imine polymer and a plurality of substituents of formula (I), or a compound comprising a poly(amidoamine) dendrimer and a plurality of substituents of formula (I)).

In some embodiments, the kit comprises the compound or the salt thereof in a container.

In some embodiments, the kit further comprises at least one of a DNA molecule, an RNA molecule, a peptide, a polypeptide, a protein, or any combinations or derivatives thereof. In some embodiments, the biomolecule is a peptide or polypeptide capable of luminescent activity. In some embodiments, the biomolecule comprises a polypeptide sequence of SEQ ID NO: 4 (LgBiT). In some embodiments, the peptide comprises a Cas9 protein. In some embodiments, the DNA molecule is a donor DNA template comprising a sequence encoding a polypeptide from SEQ ID NO: 3.

In some embodiments, the kit further comprises instructions for using the compound, or the salt thereof, for transfection of a biomolecule.

Embodiments of the present disclosure also include a method for altering a sequence of an endogenous protein in a cell, the method comprising:
assembling a ribonucleoprotein (RNP) complex comprising a Cas9 protein, a donor DNA template, and a guide RNA; and
delivering the RNP complex into a cell using a compound described herein.

Embodiments of the present disclosure also include a method for tagging an endogenous protein in a cell, the method comprising:
assembling a ribonucleoprotein (RNP) complex comprising a Cas9 protein, a donor DNA template, and a guide RNA, wherein the donor DNA template comprises a sequence encoding a peptide or polypeptide tag sequence; and
delivering the RNP complex into a cell using a compound described herein.

In some embodiments, the donor DNA template comprises a sequence encoding a peptide tag selected from SEQ ID NO: 3 and SEQ ID NO: 5. In some embodiments, the donor DNA template further comprises homology arms flanking the sequence encoding peptide or polypeptide tag sequence.

### BRIEF DESCRIPTION OF THE DRAWINGS

FIGS. 1A-1B depict results from transfection of HEK293 cells with constant molar ratio of modified PEI polymers as described in Example 2. FIG. 1A shows transfection efficiency for a NanoLuc^{®} vector as measured by NanoLuc^{®} expression, and FIG. 1B shows measurements of cell viability using CellTiter-Glo^{®} Luminescent Cell Viability Assay.
FIGS. 2A-2B depict results from transfection of HEK293 cells with modified PEI compounds in comparison to unmodified polymers and FuGENE HD as a control as described in Example 2. FIG. 2A shows the ratio of luminescence signal from NanoLuc relative to the control, and FIG. 2B shows measurements of cell viability using a CellTiter-Glo^{®} Luminescent Cell Viability Assay compared to a sample using no transfection reagent.
FIGS. 3A-3C depict results from titrations of modified PEI polymers and incubation with a constant amount of DNA before transfection of HEK cells as described in Example 2. FIG. 3A shows data from transfections carried out in the presence of serum, and FIG. 3B shows data from transfections carried out in the absence of serum. FIG. 3C shows measurements of cell viability using a CellTiter-Glo^{®} Luminescent Cell Viability Assay after transfection in the presence of serum.
FIGS. 4A-4I depict luminescence results using Nano-Glo^{®} reagent from titrations of modified PEI and PAMAM polymers and incubation with a constant amount of DNA before transfection of nine different cell types in the presence of serum, as described in Example 2.
FIG. 5A-5H show measurements of cell viability using CellTiter-Glo^{®} Luminescent Cell Viability Assay after transfection with modified PEI and PAMAM polymers in nine different cell types in the presence of serum, as described in Example 2.
FIGS. 6A-6E depict results from titrations of modified PEI and PAMAM polymers and incubation with a constant amount of DNA before transfection of five different cell types in the presence of serum, as described in Example 2.
FIG. 7A-7D show measurements of cell viability using CellTiter-Glo^{®} Luminescent Cell Viability Assay after transfection with modified PEI and PAMAM polymers in four different cell types in the presence of serum, as described in Example 2.
FIGS. 8A-8E depict results from titrations of modified PEI and PAMAM polymers and incubation with a constant amount of DNA before transfection of five different cell types in the presence of serum, as described in Example 2.
FIG. 9A-9D show measurements of cell viability using CellTiter-Glo^{®} Luminescent Cell Viability Assay after transfection with modified PEI and PAMAM polymers in five different cell types in the presence of serum, as described in Example 2.

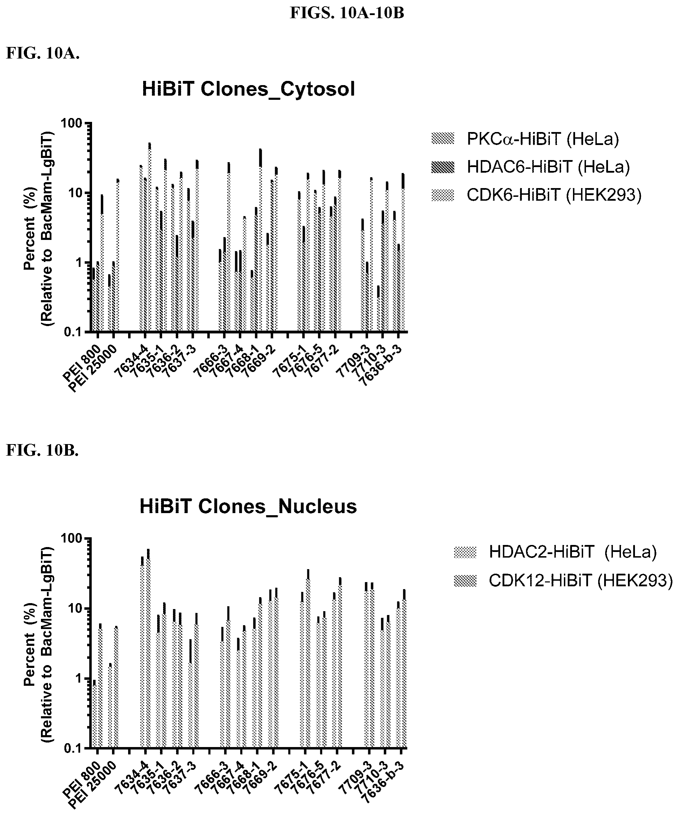

FIGS. 10A-10B depict results delivering LgBiT to the clones that stably expressed HiBiT-fusions. FIG. 10A depicts results delivering LgBiT to PKCα-HiBiT or HDAC6-HiBiT clones of HeLa cells or CDK6-HiBiT clones of HEK293 cells using the modified PEI compounds in comparison to unmodified polymers and direct transduction of BacMam CMV-LgBiT to the above corresponding clones. Percentages of luminescence signal from LgBiT-delivered PKCα-HiBiT clones, HDAC6-HiBiT, or CDK6-HiBiT clones are shown relative to the luminescence signal from the corresponding BacMam CMV-LgBiT transduced HiBiT clones for the various PEI compounds tested. FIG. 10B depicts results delivering LgBiT to HDAC2-HiBiT clones of HeLa cells or CDK12-HiBiT clones of HEK293 cells using the modified PEI compounds in comparison to unmodified polymers and direct transduction of BacMam CMV-LgBiT to the above corresponding clones. Percentages of luminescence signal from the LgBiT-delivered HDAC2-HiBiT clones of HeLa cells or CDK12-HiBiT clones of HEK293 cells are shown relative to the corresponding BacMam CMV-LgBiT transduced HiBiT-clones for the various PEI compounds tested.
FIGS. 11A-11B depict measurements of cell viability of HiBiT clones derived from HeLa and HEK293 cells after delivering LgBiT. FIG. 11A includes PKCα-HiBiT, HDAC6-HiBiT, and CDK6-HiBiT, and FIG. 11B includes HDAC2-HiBiT and CDK12-HiBiT. LgBiT delivery to these cells were either by the modified PEI compounds or the unmodified polymers. The negative control was a no PEI addition to cells. Luminescence signal (RLUs) is representative of cell viability as measured using CellTiter-Glo^{®} Luminescent Cell Viability Assay.
FIGS. 12A-12C depicts results of bioluminescence imaging of delivering LgBiT to the clones that stably expressed HDAC2-HiBiT in HeLa Cells or HDAC6-HiBiT in HeLa cells with modified PEI compounds, 7666-3 (FIG. 12A); 7668-1 (FIG. 12B); 7669-2 (FIG. 12C).
FIGS. 13A-13B depict results of screens of modified PEI compounds and unmodified PEI to examine delivery of LgBiT to different HiBiT edited cells. FIG. 13A depicts percentage of LgBiT delivery by PEI in comparison to BacMam-LgBiT transduction. FIG. 13B represents viability of cells that were treated with PEI-LgBiT complex for 24 h.
FIGS. 14A-C depict results of bioluminescence imaging of different cell lines after delivering LgBiT to HiBiT tagged proteins that are localized on the cell surface, the cytoplasm, and the nucleus.
FIGS. 15A-15C depict results of fluorescent imaging of HeLa cells after HaloTag-LgBiT delivery using modified PEIs in comparison to unmodified PEIs.
FIGS. 16A-16B depict results from the RNP delivery of a VS-HiBiT tag on the C-terminus of GAPDH in HEK293 cells using the modified PEI compound 7667-4 in comparison to FuGENE HD, ViaFect^{™}, CRISPRMax, and Nucleofection as controls. Luminescence signal (RLUs) normalized to cell number is representative of the efficiency of RNP delivery. Cell number is determined by CellTiter-Glo^{®} Luminescent Cell Viability Assay (FIG. A). Increasing the concentration of PEI shows improvement in RNP delivery, but causes more cellular toxicity (FIG. 16B).

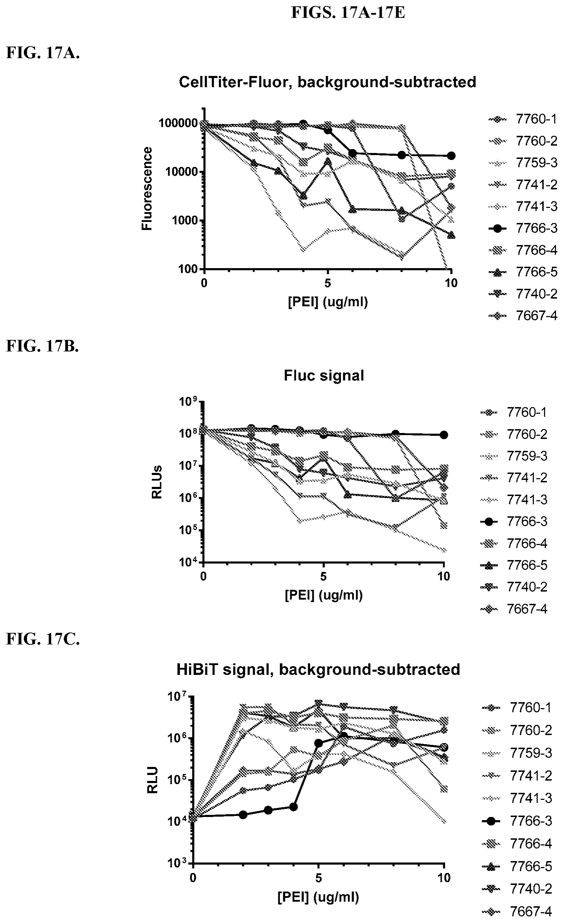

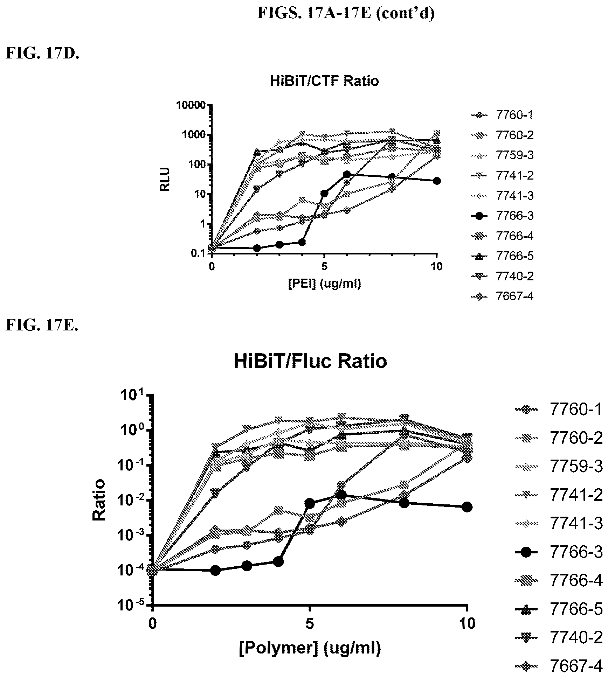

FIGS. 17A-17E depict delivery into HEK293 cells stably expressing firefly luciferase (HEK293/Fluc cells) of a constant amount of Ribonucleoprotein (RNP) complex and Single-stranded oligodeoxynucleotide (ssODN) donor template designed to insert HiBiT at the N-terminus of Fluc via CRISPR/Cas9. The RNP/ssODN mixture was delivered into the cells with a titration of modified PEI and PAMAM compounds at the listed final concentrations. Two days later, cells were measured for viability using CellTiter-Fluor (CTF; FIG. 17A), Fluc expression using ONE-Glo EX (FIG. 17B), and HiBiT signal using the HiBiT NanoDLR assay (FIG. 17C). The HiBiT signal was normalized to cell number using the HiBiT/CTF ratio (FIG. 17D) and to Fluc expression using the HiBiT/Fluc ratio (FIG. 17E).

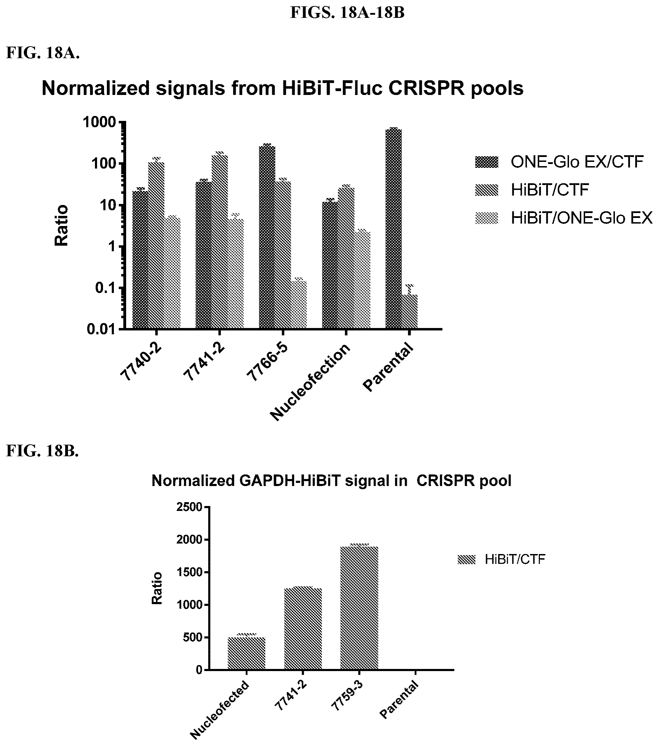

FIGS. 18A-18B depict pools of HEK293/Fluc cells in which RNP/ssODN mixtures for CRISPR knock-in of HiBiT have been delivered using either nucleofection or a modified PEI or PAMAM polymer. Cell pools were expanded for multiple days prior to measurement to eliminate complications from cell death during treatment. After knock-in of HiBiT at the N-terminus of Fluc, CellTiter-Fluor + HiBiT NanoDLR were used to measure viability, Fluc expression, and HiBiT signal (FIG. 18A). The large drop in the Fluc/CTF ratio for nucleofected cells may indicate a loss of expression caused by InDel mutations. RNP/ssODN delivery mediated by the modified polymers resulted in both higher normalized Fluc expression, but also higher normalized HiBiT signal, as indicated by the HiBiT/CTF ratio. Similarly, modified PEI polymers show higher normalized HiBiT signal compared to nucleofection after delivery of an RNP/ssODN mixture designed to knock-in HiBiT at the C-terminus of GAPDH (FIG. 18B).
FIGS. 19A-19B show results of a protein degradation assay to demonstrate that a modified PEI polymer described herein delivers functional LgBiT into cells as described in Example 6.
FIGS. 20A-20D show results of assays that differentiate intracellular and extracellular luminescence systems in four different cell lines (HEK293, A549, H1299, and Mia-PaCa2) as described in Example 7.

### DETAILED DESCRIPTION

Provided herein are modified polyethyleneimine polymers and modified poly(amidoamine) dendrimers for use in delivering biomolecules to cells.

The modified polymers and dendrimers, in some embodiments, include fluorinated substituent groups. Fluorinated compounds are both hydrophobic and lipophobic having a high phase-separation tendency in both polar and non-polar environments, and fluorination can therefore improve the affinity of polymers for cell membranes and can aid transport of molecules across the lipid bilayer of the cell membrane, as well as the endosome/lysosome membrane, facilitating endosomal escape of the polymers. In addition, fluorinated polymers have low surface energy and prefer to associate with each other at low concentrations allowing the formation of polymer complexes with nucleic acids or proteins at low concentration.

Section headings as used in this section and the entire disclosure are merely for organizational purposes and are not intended to be limiting.

### 1. Definitions

Unless otherwise defined herein, scientific and technical terms used in connection with the present disclosure shall have the meanings that are commonly understood by those of ordinary skill in the art. For example, any nomenclatures used in connection with, and techniques of, cell and tissue culture, molecular biology, immunology, microbiology, genetics and protein and nucleic acid chemistry and hybridization described herein are those that are well known and commonly used in the art. The meaning and scope of the terms should be clear; in the event, however of any latent ambiguity, definitions provided herein take precedent over any dictionary or extrinsic definition. Further, unless otherwise required by context, singular terms shall include pluralities and plural terms shall include the singular.

Definitions of specific functional groups and chemical terms are described in more detail below. For purposes of this disclosure, the chemical elements are identified in accordance with the Periodic Table of the Elements, CAS version, Handbook of Chemistry and Physics, 75^{th} Ed., inside cover, and specific functional groups are generally defined as described therein. Additionally, general principles of organic chemistry, as well as specific functional moieties and reactivity, are described in Sorrell, Organic Chemistry, 2nd edition, University Science Books, Sausalito, 2006; Smith, March's Advanced Organic Chemistry: Reactions, Mechanism, and Structure, 7th Edition, John Wiley & Sons, Inc., New York, 2013; Larock, Comprehensive Organic Transformations, 3rd Edition, John Wiley & Sons, Inc., New York, 2018; Carruthers, Some Modern Methods of Organic Synthesis, 3rd Edition, Cambridge University Press, Cambridge, 1987; the entire contents of each of which are incorporated herein by reference.

The term "alkyl," as used herein, means a straight or branched saturated hydrocarbon chain containing from 1 to 30 carbon atoms, for example 1 to 16 carbon atoms (C₁-C₁₆ alkyl), 1 to 14 carbon atoms (C₁-C₁₄ alkyl), 1 to 12 carbon atoms (C₁-C₁₂ alkyl), 1 to 10 carbon atoms (C₁-C₁₀ alkyl), 1 to 8 carbon atoms (C₁-C₈ alkyl), 1 to 6 carbon atoms (C₁-C₆ alkyl), or 1 to 4 carbon atoms (C₁-C₄ alkyl). Representative examples of alkyl include, but are not limited to, methyl, ethyl, n-propyl, iso-propyl, n-butyl, sec-butyl, iso-butyl, tert-butyl, n-pentyl, isopentyl, neopentyl, n-hexyl, 3-methylhexyl, 2,2-dimethylpentyl, 2,3-dimethylpentyl, n-heptyl, n-octyl, n-nonyl, n-decyl, n-undecyl, and n-dodecyl.

The term "aryl," as used herein, refers to a phenyl group, or a bicyclic or tricyclic aromatic fused ring system. Bicyclic fused ring systems are exemplified by a phenyl group appended to the parent molecular moiety and fused to a phenyl group. Tricyclic fused ring systems are exemplified by a phenyl group appended to the parent molecular moiety and fused to two other phenyl groups. Representative examples of bicyclic aryls include, but are not limited to, naphthyl. Representative examples of tricyclic aryls include, but are not limited to, anthracenyl.

The term "cycloalkyl" as used herein, refers to a saturated carbocyclic ring system containing three to ten carbon atoms and zero heteroatoms. The cycloalkyl may be monocyclic, bicyclic, bridged, fused, or spirocyclic. Representative examples of cycloalkyl include, but are not limited to, cyclopropyl, cyclobutyl, cyclopentyl, cyclohexyl, cycloheptyl, cyclooctyl, cyclononyl, cyclodecyl, adamantyl, bicyclo[2.2.1]heptanyl, bicyclo[3.2.1]octanyl, and bicyclo[5.2.0]nonanyl.

The term "halogen" or "halo," as used herein, means F, Cl, Br, or I.

The term "haloalkyl," as used herein, means an alkyl group, as defined herein, in which one or more hydrogen atoms are replaced by a halogen. For example, one, two, three, four, five, six, seven or eight hydrogen atoms can be replaced by a halogen. Representative examples of haloalkyl include, but are not limited to, fluoromethyl, difluoromethyl, trifluoromethyl, chloromethyl, dichloromethyl, trichloromethyl, 2-fluoroethyl, 2,2-difluoroethyl, and 2,2,2-trifluoroethyl.

The term "heteroaryl," as used herein, refers to an aromatic monocyclic ring or an aromatic bicyclic ring system or an aromatic tricyclic ring system. The aromatic monocyclic rings are five or six membered rings containing at least one heteroatom independently selected from the group consisting of N, O, and S (e.g. 1, 2, 3, or 4 heteroatoms independently selected from O, S, and N). The five-membered aromatic monocyclic rings have two double bonds, and the six membered six membered aromatic monocyclic rings have three double bonds. The bicyclic heteroaryl groups are exemplified by a monocyclic heteroaryl ring appended fused to a monocyclic aryl group, as defined herein, or a monocyclic heteroaryl group, as defined herein. The tricyclic heteroaryl groups are exemplified by a monocyclic heteroaryl ring fused to two rings independently selected from a monocyclic aryl group, as defined herein, or a monocyclic heteroaryl group as defined herein. Representative examples of monocyclic heteroaryl include, but are not limited to, pyridinyl (including pyridin-2-yl, pyridin-3-yl, pyridin-4-yl), pyrimidinyl, pyrazinyl, pyridazinyl, pyrrolyl, benzopyrazolyl, 1,2,3-triazolyl, 1,3,4-thiadiazolyl, 1,2,4-thiadiazolyl, 1,3,4-oxadiazolyl, 1,2,4-oxadiazolyl, imidazolyl, thiazolyl, isothiazolyl, thienyl, furanyl, oxazolyl, isoxazolyl, 1,2,4-triazinyl, and 1,3,5-triazinyl. Representative examples of bicyclic heteroaryl include, but are not limited to, benzimidazolyl, benzodioxolyl, benzofuranyl, benzooxadiazolyl, benzopyrazolyl, benzothiazolyl, benzothienyl, benzotriazolyl, benzoxadiazolyl, benzoxazolyl, chromenyl, imidazopyridine, imidazothiazolyl, indazolyl, indolyl, isobenzofuranyl, isoindolyl, isoquinolinyl, naphthyridinyl, purinyl, pyridoimidazolyl, quinazolinyl, quinolinyl, quinoxalinyl, thiazolopyridinyl, thiazolopyrimidinyl, thienopyrrolyl, and thienothienyl. Representative examples of tricyclic heteroaryl include, but are not limited to, dibenzofuranyl and dibenzothienyl. The monocyclic, bicyclic, and tricyclic heteroaryls are connected to the parent molecular moiety through any carbon atom or any nitrogen atom contained within the rings.

The term "heterocycle" or "heterocyclic," as used herein, means a monocyclic heterocycle, a bicyclic heterocycle, or a tricyclic heterocycle. The monocyclic heterocycle is a three-, four-, five-, six-, seven-, or eight-membered ring containing at least one heteroatom independently selected from the group consisting of O, N, and S. The three- or four-membered ring contains zero or one double bond, and one heteroatom selected from the group consisting of O, N, and S. The five-membered ring contains zero or one double bond and one, two or three heteroatoms selected from the group consisting of O, N, and S. The six-membered ring contains zero, one, or two double bonds and one, two, or three heteroatoms selected from the group consisting of O, N, and S. The seven- and eight-membered rings contains zero, one, two, or three double bonds and one, two, or three heteroatoms selected from the group consisting of O, N, and S. Representative examples of monocyclic heterocycles include, but are not limited to, azetidinyl, azepanyl, aziridinyl, diazepanyl, 1,3-dioxanyl, 1,3-dioxolanyl, 1,3-dithiolanyl, 1,3-dithianyl, imidazolinyl, imidazolidinyl, isothiazolinyl, isothiazolidinyl, isoxazolinyl, isoxazolidinyl, morpholinyl, oxadiazolinyl, oxadiazolidinyl, oxazolinyl, oxazolidinyl, oxetanyl, piperazinyl, piperidinyl, pyranyl, pyrazolinyl, pyrazolidinyl, pyrrolinyl, pyrrolidinyl, tetrahydrofuranyl, tetrahydropyranyl, tetrahydropyridinyl, tetrahydrothienyl, thiadiazolinyl, thiadiazolidinyl, 1,2-thiazinanyl, 1,3-thiazinanyl, thiazolinyl, thiazolidinyl, thiomorpholinyl, 1,1-dioxidothiomorpholinyl (thiomorpholine sulfone), thiopyranyl, and trithianyl. The bicyclic heterocycle is a monocyclic heterocycle fused to a phenyl group, or a monocyclic heterocycle fused to a monocyclic cycloalkyl, or a monocyclic heterocycle fused to a monocyclic cycloalkenyl, or a monocyclic heterocycle fused to a monocyclic heterocycle, or a spiro heterocycle group, or a bridged monocyclic heterocycle ring system in which two non-adjacent atoms of the ring are linked by an alkylene bridge of 1, 2, 3, or 4 carbon atoms, or an alkenylene bridge of two, three, or four carbon atoms. Representative examples of bicyclic heterocycles include, but are not limited to, benzopyranyl, benzothiopyranyl, chromanyl, 2,3-dihydrobenzofuranyl, 2,3-dihydrobenzothienyl, 2,3-dihydroisoquinoline, 2-azaspiro[3.3]heptan-2-yl, azabicyclo[2.2.1]heptyl (including 2-azabicyclo[2.2.1]hept-2-yl), 2,3-dihydro-1H-indolyl, isoindolinyl, octahydrocyclopenta[c]pyrrolyl, octahydropyrrolopyridinyl, and tetrahydroisoquinolinyl. Tricyclic heterocycles are exemplified by a bicyclic heterocycle fused to a phenyl group, or a bicyclic heterocycle fused to a monocyclic cycloalkyl, or a bicyclic heterocycle fused to a monocyclic cycloalkenyl, or a bicyclic heterocycle fused to a monocyclic heterocycle, or a bicyclic heterocycle in which two non-adjacent atoms of the bicyclic ring are linked by an alkylene bridge of 1, 2, 3, or 4 carbon atoms, or an alkenylene bridge of two, three, or four carbon atoms. Examples of tricyclic heterocycles include, but are not limited to, octahydro-2,5-epoxypentalene, hexahydro-2H-2,5-methanocyclopenta[b]furan, hexahydro-1H-1,4-methanocyclopenta[c]furan, aza-adamantane (1-azatricyclo[3.3.1.1^{3,7}]decane), and oxa-adamantane (2-oxatricyclo[3.3.1.1^{3,7}]decane). The monocyclic, bicyclic, and tricyclic heterocycles are connected to the parent molecular moiety through any carbon atom or any nitrogen atom contained within the rings.

The term "perfluoroalkyl," as used herein, refers to an alkyl group in which each hydrogen is replaced with fluorine. Representative examples of perfluoroalkyl include, but are not limited to, trifluoromethyl, perfluoroethyl, perfluoropropyl, perfluorobutyl, perfluoropentyl, and perfluorohexyl.

The term "poly(amidoamine)" (or "PAMAM"), as used herein, refers to an art-recognized class of dendrimer with a diamine core and repetitively branched subunits having amide and amine functional groups generated by reaction of the diamine with methyl acrylate and then another diamine. These dendrimers are defined by the number of "layers" of amidoamine groups that extend from the core with each "layer" referenced as a "generation." For example, ethylenediamine can be reacted with methyl acrylate to generate methyl 3-[2-[bis(3-methoxy-3-oxopropyl)amino]ethyl-(3-methoxy-3-oxopropyl)amino]propanoate, which can then be reacted with more ethylenediamine to form the "Generation 0" PAMAM, N-(2-aminoethyl)-3-[[3-(2-aminoethylamino)-3-oxopropyl]-[2-[bis[3-(2-aminoethylamino)-3-oxopropyl]amino]ethyl]amino]propanamide. Repeating the same sequence of reactions to similarly functionalize each primary amino group of the Generation 0 PAMAM can form the Generation 1 PAMAM, and so on. The chemical structure of a Generation 1 PAMAM, with the "core" Generation 0 PAMAM structure highlighted, is shown below:

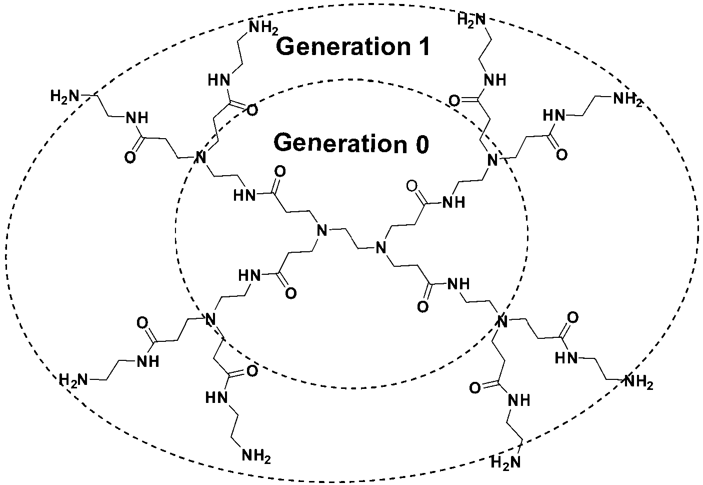

The term "polyethyleneimine" (or "PEI"), as used herein, refers to a polymer based on repeating iminoethylene groups. A "linear polyethyleneimine" is a linear polymer having a formula -(CH₂-CH₂-NH)ₙ- and thus includes only secondary amino groups within the polymer chain. A "branched polyethyleneimine" is a branched polymer that is synthesized by ring-opening polymerization of aziridine and includes primary, secondary, and tertiary amino groups. Branched polyethyleneimines are often depicted as illustrated below, although one skilled in the art will appreciate that branched polyethyleneimines are not polymers having repeat units of the exact type shown between the brackets; rather, this merely provides an illustration of the different ways in which the individual -CH₂-CH₂-NH- groups may be linked together, with additional linkages and branch points being possible:

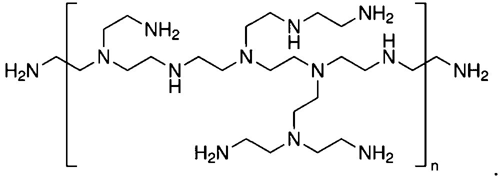

In some instances, the number of carbon atoms in a group (e.g., alkyl, haloalkyl, or cycloalkyl) is indicated by the prefix "Cₓ-C_{y}-", wherein x is the minimum and y is the maximum number of carbon atoms in the group. Thus, for example, "C₁-C₃-alkyl" refers to an alkyl group containing from 1 to 3 carbon atoms, and "C₁-C₆-haloalkyl" refers to a haloalkyl group containing from 1 to 6 carbon atoms.

The term "substituent" refers to a group substituted on an atom of the indicated group.

When a group or moiety can be substituted, the term "substituted" indicates that one or more (e.g., 1, 2, 3, 4, 5, or 6; in some embodiments 1, 2, or 3; and in other embodiments 1 or 2) hydrogens on the group indicated in the expression using "substituted" can be replaced with a selection of recited indicated groups or with a suitable group known to those of skill in the art (e.g., one or more of the groups recited below). Substituent groups include, but are not limited to, halogen, =O, =S, cyano, nitro, fluoroalkyl, alkoxyfluoroalkyl, fluoroalkoxy, alkyl, alkenyl, alkynyl, haloalkyl, haloalkoxy, heteroalkyl, cycloalkyl, cycloalkenyl, aryl, heteroaryl, heterocycle, cycloalkylalkyl, heteroarylalkyl, arylalkyl, hydroxy, hydroxyalkyl, alkoxy, alkoxyalkyl, alkylene, aryloxy, phenoxy, benzyloxy, amino, alkylamino, acylamino, aminoalkyl, arylamino, sulfonylamino, sulfinylamino, sulfonyl, alkylsulfonyl, arylsulfonyl, aminosulfonyl, sulfinyl,-COOH, ketone, amide, carbamate, and acyl.

As used herein, the terms *"Oplophorus* luciferase" and *"Oplophorus-derived* luciferase" are used interchangeably and refer to a luciferase secreted from the deep-sea shrimp *Oplophorus gracilirostris* (e.g., SEQ ID NO: 1) including wild-type, variants, and mutants thereof. For example, suitable Oplophorus luciferase variants are described in U.S. Pat. Nos. 8,557,970 and 8,669,103, each of which is incorporated herein by reference in its entirety. Exemplary Oplophorus-derived luciferases include, for example, that of SEQ ID NO: 2 (also interchangeably referred to herein as "NanoLuc," "Nluc," "Nluc luciferase," and "Nluc enzyme").

For compounds described herein, groups and substituents thereof may be selected in accordance with permitted valence of the atoms and the substituents such that the selections and substitutions result in a stable compound, e.g., which does not spontaneously undergo transformation such as by rearrangement, cyclization, elimination, etc.

The terms "comprise(s)," "include(s)," "having," "has," "can," "contain(s)," and variants thereof, as used herein, are intended to be open-ended transitional phrases, terms, or words that do not preclude the possibility of additional acts or structures. The singular forms "a," "and" and "the" include plural references unless the context clearly dictates otherwise. Many embodiments herein are described using open "comprising" language. Such embodiments encompass multiple closed "consisting of" and/or "consisting essentially of" embodiments, which may alternatively be claimed or described using such language. The present disclosure also contemplates other embodiments "comprising," "consisting of" and "consisting essentially of," the embodiments or elements presented herein, whether explicitly set forth or not.

For the recitation of numeric ranges herein, each intervening number there between with the same degree of precision is explicitly contemplated. For example, for the range of 6-9, the numbers 7 and 8 are contemplated in addition to 6 and 9, and for the range 6.0-7.0, the number 6.0, 6.1, 6.2, 6.3, 6.4, 6.5, 6.6, 6.7, 6.8, 6.9, and 7.0 are explicitly contemplated.

### 2. Polymers

The present disclosure includes modified polyamine polymers that are suitable for delivery of biomolecules into cells, such as genes, proteins, and ribonucleoproteins and related complexes.

In some embodiments, the disclosure provides a compound comprising:
a polyethyleneimine polymer; and
a plurality of substituents bound to amino groups of the polyethyleneimine polymer,
wherein each substituent independently has a formula (I):

-X-(CH₂)ₙ-Z (I),

wherein:
X is a bond or -C(O)-O-;
n is 0, 1, 2, 3, 4, or 5; and
Z is selected from a haloalkyl group, an aryl group, a substituted aryl group, a heteroaryl group, and a substituted heteroaryl group.

In some embodiments, the polyethyleneimine polymer has a weight average molecular weight of about 500 Da to about 250000 Da, or about 500 Da to about 30000 Da, or about 500 Da to about 2000 Da, or about 5000 Da to about 25000 Da. For example, the polyethyleneimine polymer may have a weight average molecular weight of about 500 Da, about 600 Da, about 700 Da, about 800 Da, about 900 Da, about 1000 Da, about 1200 Da, about 1400 Da, about 1600 Da, about 1200 Da, about 1400 Da, about 1600 Da, about 1800 Da, about 2000 Da, about 3000 Da, about 4000 Da, about 5000 Da, about 6000 Da, about 7000 Da, about 8000 Da, about 9000 Da, about 10000 Da, about 15000 Da, about 20000 Da, about 25000 Da, about 30000 Da, about 35000 Da, about 40000 Da, about 45000 Da, about 50000 Da, about 100000 Da, about 150000 Da, about 200000 Da, or about 250000 Da. In particular embodiments, the polyethyleneimine polymer has a weight average molecular weight of about 600 Da, about 800 Da, about 1200 Da, about 1800 Da, about 5000 Da, about 10000 Da, or about 25000 Da.

The polyethyleneimine polymer may be linear or branched. In some embodiments, the polyethyleneimine polymer is a linear polyethyleneimine polymer. In some embodiments, the polyethyleneimine polymer is a branched polyethyleneimine polymer.

In some embodiments, the polyethyleneimine polymer may be cross-linked. For example, the polyethyleneimine polymer, before or after functionalization such that the polymer includes a plurality of substituents of formula (I), can be cross-linked with urea (i.e. such that two amino groups on two different polymers are linked together by a C=O group).

In the plurality of substituents of formula (I), X is a bond or -C(O)-O-. In some embodiments, X is a bond. In some embodiments, X is -C(O)-O-.

In the plurality of substituents of formula (I), n is 0, 1, 2, 3, 4, or 5. In some embodiments, n is 0. In some embodiments, n is 1. In some embodiments, n is 2. In some embodiments, n is 3. In some embodiments, n is 4. In some embodiments, n is 5. In some embodiments, n is 0, 1, or 2. In some embodiments, n is 1 or 2.

In some embodiments, X is -C(O)-O- and n is 1 or 2. In some embodiments, X is a bond and n is 1 or 2.

In the plurality of substituents of formula (I), Z is selected from a haloalkyl group, an aryl group, a substituted aryl group, a heteroaryl group, and a substituted heteroaryl group. In some embodiments, Z is selected from a haloalkyl group, a substituted aryl group, and an unsubstituted heteroaryl group.

In some embodiments, Z is a haloalkyl group. In some embodiments, the haloalkyl group is a perfluoroalkyl group. In some embodiments, Z is a haloalkyl group having the formula -(CF₂)ₘ-CF₃, wherein m is 1, 2, 3, 4, 5, 6, 7, 8, 9, or 10. For example, in some embodiments, m is 1. In some embodiments, m is 2. In some embodiments, m is 3. In some embodiments, m is 4. In some embodiments, m is 5. In some embodiments, m is 6. In some embodiments, m is 7. In some embodiments, m is 8. In some embodiments, m is 9. In some embodiments, m is 10. In some embodiments, m is 3, 4, 5, 6, or 7.

In some embodiments, Z is an aryl group or a substituted aryl group. In some embodiments, Z is a substituted aryl group. In some embodiments, Z is a substituted phenyl group. In some embodiments, Z is a phenyl group substituted with 1, 2, 3, 4, or 5 substituents selected from halo. In some embodiments, Z is a pentafluorophenyl group.

In some embodiments, Z is a heteroaryl group or a substituted heteroaryl group. In some embodiments, Z is an unsubstituted heteroaryl group. In some embodiments, Z is an unsubstituted monocyclic heteroaryl group. In some embodiments, the heteroaryl group is a monocyclic heteroaryl group having 1, 2, or 3 heteroatoms independently selected from N, O, and S. In some embodiments, Z is an unsubstituted pyridyl group.

In some embodiments, X is -C(O)O-, and n is 1 or 2. In some embodiments, X is a bond, and n is 1 or 2.

In some embodiments, about 0.1 mol% to about 60 mol% of the amino groups of the polyethyleneimine polymer are bound to a substituent of formula (I). For example, about 10 mol% to about 60 mol%, about 5 mol% to about 50 mol%, or about 8 mol% to about 40 mol% of the amino groups of the polyethyleneimine polymer are bound to a substituent of formula (I). In some embodiments, about 0.1 mol%, about 0.5 mol%, about 1 mol%, about 2 mol%, about 5 mol%, about 6 mol%, about 7 mol%, about 8 mol%, about 9 mol%, about 10 mol%, about 15 mol%, about 20 mol%, about 25 mol%, about 30 mol%, about 35 mol%, about 40 mol%, about 45 mol%, about 50 mol%, about 55 mol%, about 60 mol%, about 65 mol%, about 70 mol%, about 75 mol%, or about 80 mol% of the amino groups of the polyethyleneimine polymer are bound to a substituent of formula (I). The degree of modification can be tuned, for example, by using different amounts of reagents as further discussed below. The degree of modification can be determined, for example, using ¹H NMR spectroscopy.

In some embodiments, the disclosure provides a compound comprising:
a poly(amidoamine) dendrimer; and
a plurality of substituents bound to amino groups of the poly(amidoamine) dendrimer,
wherein each substituent independently has a formula (I):

-X-(CH₂)ₙ-Z (I),

wherein:
X is a bond or -C(O)-O-;
n is 0, 1 or 2; and
Z is selected from a haloalkyl group, an aryl group, a substituted aryl, a heteroaryl group, and a substituted heteroaryl group.

In some embodiments, the poly(amidoamine) dendrimer is a Generation 0, Generation 1, Generation 2, Generation 3, Generation 4, Generation 5, Generation 6, Generation 7, Generation 8, Generation 9, or Generation 10 poly(amidoamine) dendrimer. In some embodiments, the poly(amidoamine) dendrimer is a Generation 1, Generation 2, Generation 3, Generation 4, Generation 5, Generation 6, Generation 7, Generation 8, Generation 9, or Generation 10 poly(amidoamine) dendrimer. In some embodiments, the poly(amidoamine) dendrimer is a Generation 1, Generation 2, Generation 3, or Generation 4 poly(amidoamine) dendrimer. In some embodiments, the poly(amidoamine) dendrimer is a Generation 1 poly(amidoamine) dendrimer. In some embodiments, the poly(amidoamine) dendrimer is a Generation 2 poly(amidoamine) dendrimer. In some embodiments, the poly(amidoamine) dendrimer is a Generation 3 poly(amidoamine) dendrimer. In some embodiments, the poly(amidoamine) dendrimer is a Generation 4 poly(amidoamine) dendrimer.

In the plurality of substituents of formula (I), X is a bond or -C(O)-O-. In some embodiments, X is a bond. In some embodiments, X is -C(O)-O-.

In the plurality of substituents of formula (I), n is 0, 1, 2, 3, 4, or 5. In some embodiments, n is 0. In some embodiments, n is 1. In some embodiments, n is 2. In some embodiments, n is 3. In some embodiments, n is 4. In some embodiments, n is 5. In some embodiments, n is 0, 1, or 2. In some embodiments, n is 1 or 2.

In some embodiments, X is a bond and n is 1.

In the plurality of substituents of formula (I), Z is selected from a haloalkyl group, an aryl group, a substituted aryl group, a heteroaryl group, and a substituted heteroaryl group. In some embodiments, Z is selected from a haloalkyl group, a substituted aryl group, and an unsubstituted heteroaryl group.

In some embodiments, Z is a haloalkyl group. In some embodiments, the haloalkyl group is a perfluoroalkyl group. In some embodiments, Z is a haloalkyl group having the formula -(CF₂)ₘ-CF₃, wherein m is 1, 2, 3, 4, 5, 6, 7, 8, 9, or 10. For example, in some embodiments, m is 1. In some embodiments, m is 2. In some embodiments, m is 3. In some embodiments, m is 4. In some embodiments, m is 5. In some embodiments, m is 6. In some embodiments, m is 7. In some embodiments, m is 8. In some embodiments, m is 9. In some embodiments, m is 10. In some embodiments, m is 3, 4, 5, 6, or 7.

In some embodiments, Z is an aryl group or a substituted aryl group. In some embodiments, Z is a substituted aryl group. In some embodiments, Z is a substituted phenyl group. In some embodiments, Z is a phenyl group substituted with 1, 2, 3, 4, or 5 substituents selected from halo. In some embodiments, Z is a pentafluorophenyl group.

In some embodiments, Z is a heteroaryl group or a substituted heteroaryl group. In some embodiments, Z is an unsubstituted heteroaryl group. In some embodiments, Z is an unsubstituted monocyclic heteroaryl group. In some embodiments, the heteroaryl group is a monocyclic heteroaryl group having 1, 2, or 3 heteroatoms independently selected from N, O, and S. In some embodiments, Z is an unsubstituted pyridyl group.

In some embodiments, X is -C(O)O-, and n is 1 or 2. In some embodiments, X is a bond, and n is 1 or 2.

In some embodiments, about 0.1 mol% to about 80 mol% of the primary amino groups of the poly(amidoamine) dendrimer are bound to a substituent of formula (I). For example, about 10 mol% to about 70 mol%, or about 20 mol% to about 70 mol% of the primary amino groups of the poly(amidoamine) dendrimer are bound to a substituent of formula (I). In some embodiments, about 0.1 mol%, about 0.5 mol%, about 1 mol%, about 2 mol%, about 5 mol%, about 6 mol%, about 7 mol%, about 8 mol%, about 9 mol%, about 10 mol%, about 15 mol%, about 20 mol%, about 25 mol%, about 30 mol%, about 35 mol%, about 40 mol%, about 45 mol%, about 50 mol%, about 55 mol%, about 60 mol%, about 65 mol%, about 70 mol%, about 75 mol%, or about 80 mol% of the primary amino groups of the poly(amidoamine) dendrimer are bound to a substituent of formula (I). The degree of modification can be tuned, for example, by using different amounts of reagents as further discussed below. The degree of modification can be determined, for example, using ¹H NMR spectroscopy.

The polymers include functional groups that may be cationic (e.g., -NH₂ may be -NH₃⁺), and thus in some embodiments, the salts may be formed with a suitable anion. Examples of suitable inorganic anions include, but are not limited to, those derived from the following inorganic acids: hydrochloric, hydrobromic, hydroiodic, sulfuric, sulfurous, nitric, nitrous, phosphoric, and phosphorous. Examples of suitable organic anions include, but are not limited to, those derived from the following organic acids: 2-acetyoxybenzoic, acetic, ascorbic, aspartic, benzoic, camphorsulfonic, cinnamic, citric, edetic, ethanedisulfonic, ethanesulfonic, fumaric, glucoheptonic, gluconic, glutamic, glycolic, hydroxymaleic, hydroxynaphthalene carboxylic, isethionic, lactic, lactobionic, lauric, maleic, malic, methanesulfonic, mucic, oleic, oxalic, palmitic, pamoic, pantothenic, phenylacetic, phenylsulfonic, propionic, pyruvic, salicylic, stearic, succinic, sulfanilic, tartaric, toluenesulfonic, and valeric. In some embodiments, the compound is a hydrochloride salt.

The compounds be prepared by a variety of methods. For example, carbamate-modified linear or branched PEI polymers or PAMAM dendrimers can be prepared by reacting with fluorinated alkyl p-nitrobenzene carbonate compounds as illustrated in Schemes 1A, 1B, and 1C. PEI polymers and PAMAM dendrimers can also be modified by reacting with fluorinated aldehydes to form the imine adducts and followed by imine reduction with NaBH₄ to give the desired alkylated polymers as illustrated in Scheme 2. The amine modification percentages can be tuned by adding different amounts of reagents. In addition, the primary amines of branched PEIs can be protected by BOC in a certain degree and followed by alkylation with fluorinated alkyl halide and deprotection to yield the free primary amine alkylated PEI compounds as illustrated in Scheme 3.

In the schemes that follow, the following abbreviations are used: Boc is *tert-*butyloxycarbonyl; DCM is dichloromethane; THF is tetrahydrofuran; TFA is trifluoroacetic acid; and TIPS is triisopropylsilane.

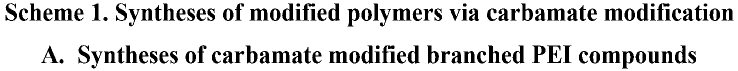

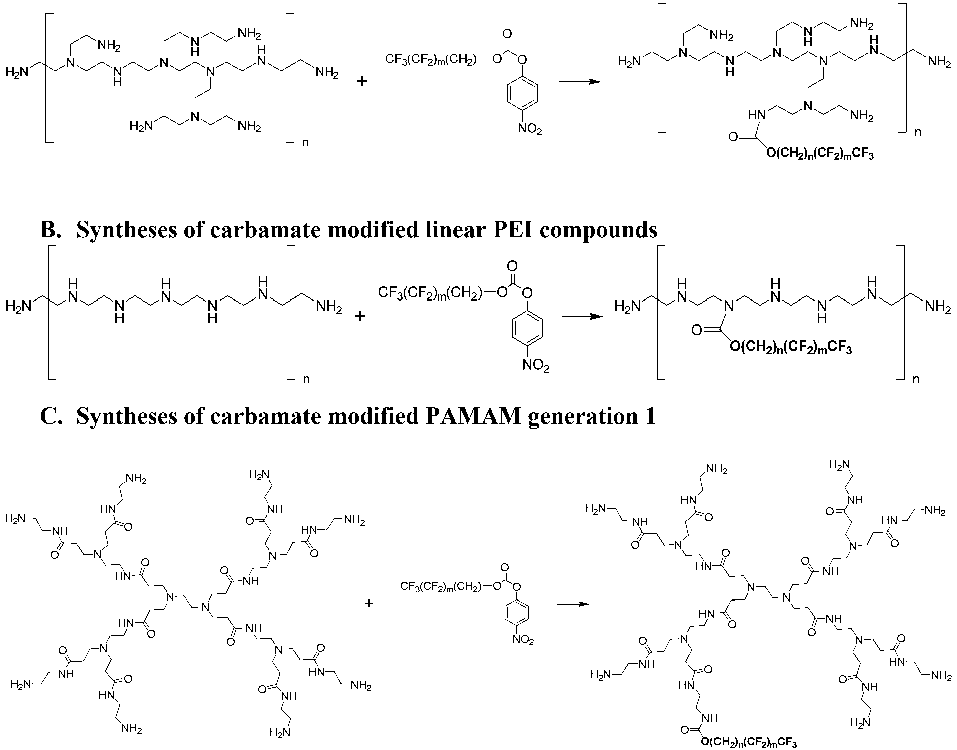

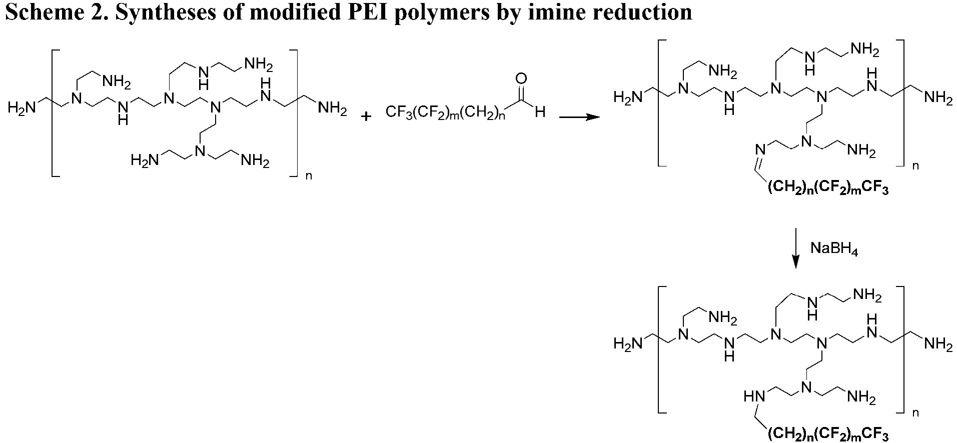

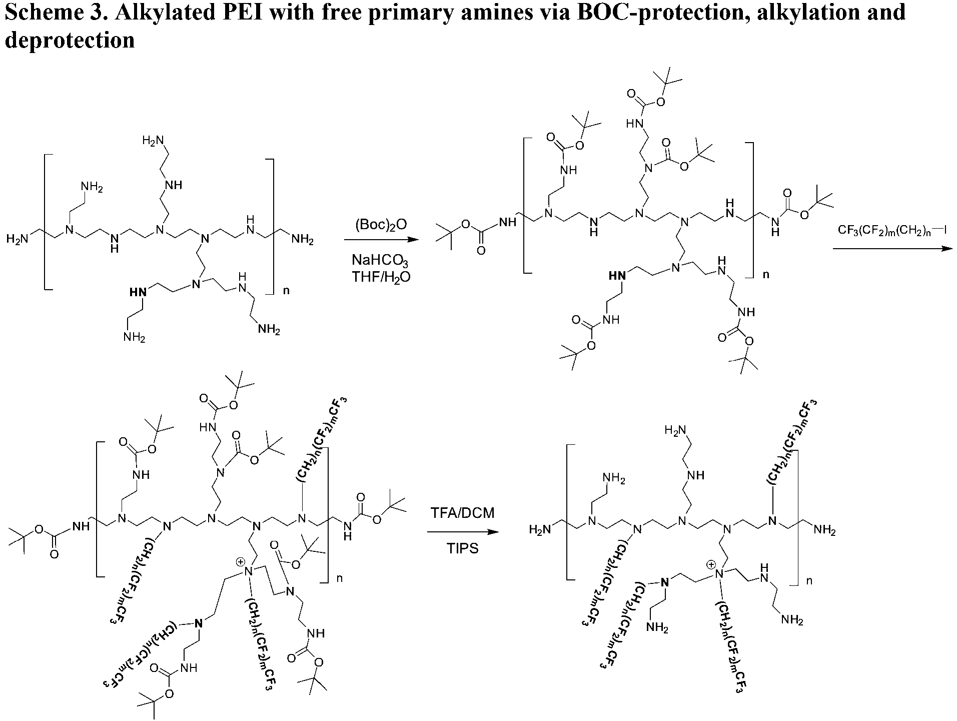

The compounds and intermediates herein may be isolated and purified by methods well-known to those skilled in the art of organic synthesis. Examples of conventional methods for isolating and purifying compounds can include, but are not limited to, chromatography on solid supports such as silica gel, alumina, or silica derivatized with alkylsilane groups, by recrystallization at high or low temperature with an optional pretreatment with activated carbon, thin-layer chromatography, distillation at various pressures, sublimation under vacuum, and trituration, as described for instance in "Vogel's Textbook of Practical Organic Chemistry," 5th edition (1989), by Furniss, Hannaford, Smith, and Tatchell, pub. Longman Scientific & Technical, Essex CM20 2JE, England.

Reaction conditions and reaction times for each individual step can vary depending on the particular reactants employed and substituents present in the reactants used. Specific procedures are provided in the Examples section. Reactions can be worked up in the conventional manner, e.g., by eliminating the solvent from the residue and further purified according to methodologies generally known in the art such as, but not limited to, crystallization, distillation, extraction, trituration, and chromatography. Unless otherwise described, the starting materials and reagents are either commercially available or can be prepared by one skilled in the art from commercially available materials using methods described in the chemical literature. Starting materials, if not commercially available, can be prepared by procedures selected from standard organic chemical techniques, techniques that are analogous to the synthesis of known, structurally similar compounds, or techniques that are analogous to the above described schemes or the procedures described in the synthetic examples section.

Routine experimentations, including appropriate manipulation of the reaction conditions, reagents and sequence of the synthetic route, protection of any chemical functionality that cannot be compatible with the reaction conditions, and deprotection at a suitable point in the reaction sequence of the method are included in the scope of the invention. Suitable protecting groups and the methods for protecting and deprotecting different substituents using such suitable protecting groups are well known to those skilled in the art; examples of which can be found in the treatise by PGM Wuts entitled "Greene's Protective Groups in Organic Synthesis" (5th ed.), John Wiley & Sons, Inc. (2014), which is incorporated herein by reference in its entirety. Synthesis of the compounds of the invention can be accomplished by methods analogous to those described in the synthetic schemes described hereinabove and in specific examples.

The synthetic schemes and specific examples as described are illustrative and are not to be read as limiting the scope of the invention as it is defined in the claims. All alternatives, modifications, and equivalents of the synthetic methods and specific examples are included within the scope of the claims.

Exemplary polymers that have been prepared include those listed in Table 1. Further details regarding the syntheses of these polymers, including specific examples with actual and/or theoretical modification percentages, can be found in the Examples

**Table 1. Exemplary Polymers**

| **Compound** | **Polymer** | **Modification Tail** |
|---|---|---|
| 7668(WZ-0856) | PEI 25000 | -C(O)O-(CH₂)₂(CF₂)₅CF₃ |
| 7675(WZ-0857) | PEI 800 | -C(O)O-(CH₂)₂(CF₂)₅CF₃ |
| 7666 (WZ-0853) | PEI 25000 | -C(O)O-(CH₂)₂(CF₂)₃CF₃ |
| 7667 (WZ-0852) | PEI 800 | -C(O)O-(CH₂)₂(CF₂)₃CF₃ |
| 7729 (WZ-0882) | PEI 600 | -C(O)O-(CH₂)₂(CF₂)₃CF₃ |
| 7730(WZ-0885) | PEI 1200 | -C(O)O-(CH₂)₂(CF₂)₃CF₃ |
| 7731 (WZ-0883) | PEI 1800 | -C(O)O-(CH₂)₂(CF₂)₃CF₃ |
| 7732 (WZ-0884) | PEI 10000 | -C(O)O-(CH₂)₂(CF₂)₃CF₃ |
| 7738 (wz-0891) | PEI 600 | -C(O)O-(CH₂)₂(CF₂)₂CF₃ |
| 7739 (wz-0892) | PEI 800 | -C(O)O-(CH₂)₂(CF₂)₂CF₃ |
| 7740 (wz-0893) | PEI 1200 | -C(O)O-(CH₂)₂(CF₂)₂CF₃ |
| 7741 (wz-0894) | PEI 1800 | -C(O)O-(CH₂)₂(CF₂)₂CF₃ |
| 7782 (wz-0923) | PEI 10000 | -C(O)O-(CH₂)₂(CF₂)₂CF₃ |
| 7783 (wz-0924) | PEI 25000 | -C(O)O-(CH₂)₂(CF₂)₂CF₃ |
| 7742 (wz-0895) | PEI 600 | -C(O)O-(CH₂)₂CF₂CF₃ |
| 7743 (wz-0896) | PEI 800 | -C(O)O-(CH₂)₂CF₂CF₃ |
| 7744 (wz-0897) | PEI 1200 | -C(O)O-(CH₂)₂CF₂CF₃ |
| 7745 (wz-0898) | PEI 1800 | -C(O)O-(CH₂)₂CF₂CF₃ |
| 7758 (wz-0902) | PEI 600 | -C(O)O-CH₂(CF₂)₂CF₃ |
| 7759 (wz-0903) | PEI 800 | -C(O)O-CH₂(CF₂)₂CF₃ |
| 7760 (wz-0904) | PEI 1200 | -C(O)O-CH₂(CF₂)₂CF₃ |
| 7766(WZ-0908) | PAMAM G1 | -C(O)O-(CH₂)₂(CF₂)₂CF₃ |
| 7767(WZ-0909) | PAMAM G3 | -C(O)O-(CH₂)₂(CF₂)₂CF₃ |
| 7768(WZ-0910) | PAMAM G5 | -C(O)O-(CH₂)₂(CF₂)₂CF₃ |
| 7825(WZ-0949) | PAMAM G2 | -C(O)O-(CH₂)₂(CF₂)₂CF₃ |
| 7826(WZ-0950) | PAMAM G7 | -C(O)O-(CH₂)₂(CF₂)₂CF₃ |
| 7829(WZ-0952) | PAMAM G4 | -C(O)O-(CH₂)₂(CF₂)₂CF₃ |
| 7830(WZ-0953) | PAMAM G6 | -C(O)O-(CH₂)₂(CF₂)₂CF₃ |
| 7838(WZ-0957) | Linear PEI-10K | -C(O)O-(CH₂)₂(CF₂)₃CF₃ |
| 7839(WZ-0958) | Linear PEI-10K | -C(O)O-(CH₂)₂(CF₂)₂CF₃ |
| 7840(WZ-0961) | Linear PEI-25K | -C(O)O-(CH₂)₂(CF₂)₃CF₃ |
| 7841(WZ-0962) | Linear PEI-25K | -C(O)O-(CH₂)₂(CF₂)₂CF₃ |
| 7842(WZ-0963) | Linear PEI-2.5K | -C(O)O-(CH₂)₂(CF₂)₃CF₃ |
| 7843(WZ-0964) | Linear PEI-250K | -C(O)O-(CH₂)₂(CF₂)₃CF₃ |
| 7636(WZ-0867) | PEI 800 | -(CH2)2(CF2)5CF3 |
| 7637(WZ-0868) | PEI 25000 | -(CH2)2(CF2)5CF3 |
| 7709(WZ-0869) | PEI 800 | -(CH2)2(CF2)7CF3 |
| 7710(WZ-0870) | PEI 25000 | -(CH2)2(CF2)7CF3 |
| 7669(WZ-0860) | PEI-25K | Pentafluorobenzyl |
| 7677(WZ-0861) | PEI-800 | Pentafluorobenzyl |
| 7676(WZ-0862) | PEI-25K | CH₂Py |
| 7827(WZ-0954) | PEI-1200 | -CH₂(CF₂)₃CF₃ |
| 7833(WZ-0955) | PEI-25000 | -CH₂(CF₂)₃CF₃ |

### 3. Methods

Embodiments of the present disclosure include various compositions and methods used to deliver a biomolecule-of-interest into a cell. In accordance with these embodiments, the compositions and methods include the use of modified polyamine polymers, such as PEIs and PAMAM dendrimers, to deliver one or more of a deoxyribonucleic acid (DNA) molecule, a ribonucleic acid (RNA) molecule, a peptide, a polypeptide, a protein, a ribonucleoprotein, or any combinations or derivatives thereof into a cell, and in some cases, without the need for electroporation.

Compositions comprising PEIs or PAMAM dendrimers, or any combination thereof, as disclosed herein, can be used to deliver a nucleic acid molecule into a cell (e.g., gene transfection). In some embodiments, the nucleic acid is a polynucleotide comprising DNA, RNA, or combinations and derivatives thereof. The terms "nucleic acid molecule," "nucleic acid sequence," and "polynucleotide" are synonymous and are intended to encompass a polymer of DNA or RNA, which can be single-stranded or double-stranded, and which can contain non-natural or altered nucleotides. The terms include, as equivalents, analogs of either RNA or DNA made from nucleotide analogs and modified polynucleotides such as, though not limited to, methylated and/or capped polynucleotides. Nucleic acids are typically linked via phosphate bonds to form nucleic acid sequences or polynucleotides, though many other linkages are known in the art (e.g., phosphorothioates, boranophosphates, and the like).

The one or more nucleic acid molecules may be DNA, RNA, or combinations thereof (e.g., a DNA/RNA hybrid). In some embodiments, the nucleic acid molecule is a plasmid. The term "plasmid," as used herein, refers to a small DNA molecule within a cell that is physically separated from a chromosomal DNA and can replicate independently (i.e. as an "episome"). Plasmids occur naturally in bacteria, archaea, and other eukaryotic organisms and commonly exist as small circular double-stranded DNA molecules. Synthetic plasmids are widely used in the art as vectors in molecular cloning, driving the replication of recombinant DNA sequences within host organisms. Plasmid DNA may be generated using routine molecular biology techniques such as those described in, e.g., Green and Sambrook, Molecular Cloning: A Laboratory Manual (Fourth Edition), Cold Spring Harbor Laboratory Press (June 15, 2012), or may be obtained from commercial sources.

The plasmid may be any suitable recombinant DNA or RNA plasmid that comprises a heterologous nucleic acid sequence to be delivered to a target cell, either *in vitro* or *in vivo.* The heterologous nucleic acid sequence may encode a gene product (e.g., a protein) of interest for the purposes of, for example, disease treatment or prevention, and may optionally be in the form of an expression cassette. The term "recombinant" refers to a polynucleotide of semisynthetic, or synthetic origin, which either does not occur in nature or is linked to another polynucleotide in an arrangement not found in nature. The term "heterologous," as used herein, refers to a nucleic acid sequence obtained or derived from a genetically distinct entity from the rest of the entity to which it is being compared.

In some embodiments, PEIs or PAMAM dendrimers, as disclosed herein, can be used to deliver proteins, peptides, or antibodies into a cell. For example, PEIs or PAMAM dendrimers can be used to deliver proteins or peptides (or polynucleotides thereof) capable of forming a bioluminescent complex (e.g., complementary), such as, but not limited to, NanoLuc (SEQ ID NO: 2), HiBiT (SEQ ID NO: 3), LgBiT (SEQ ID NO: 4), SmBiT (SEQ ID NO: 5), DarkBiT (SEQ ID NO: 6), DarkBiT (SEQ ID NO: 7; SEQ ID NO; 8), LgTrip 3092 (SEQ ID NO: 9), LgTrip 3546 (SEQ ID NO: 10), LgTrip 2098 (SEQ ID NO: 11), and SmTrip9 (SEQ ID NO: 12). In some embodiments, complementary bioluminescent proteins or peptides can be used to tag one or more proteins-of-interest or peptides-of-interest for subsequent assessment or monitoring based on the detection or non-detection of bioluminescence (e.g., formation of the bioluminescent complex).

In accordance with these embodiments, the delivered proteins or peptides using modified PEIs or PAMAM dendrimers can be used to study protein-protein interactions, protein interference with blocking antibodies, intracellular trafficking, and protein or peptide biological functions. For example, the delivered peptides or proteins using modified PEIs or PAMAM dendrimers can form the complementary proteins with the other fragment-tagged target protein for protein quantification in living cells. In one embodiment, HiBiT-tagging protein targets (e.g., proteins-of-interest comprising a HiBiT tag) can be quantified by directly delivering LgBiT in cells, obviating the need for a separate gene transfection step (e.g., BacMam transfection, nucleofection, etc.).

In some embodiments, the nucleic acid molecule is a DNA plasmid that comprises one or more nucleic acid sequences that mediate editing or modification of a target gene. For example, the DNA plasmid may comprise components of the CRISPR/Cas9 gene editing system, including but not limited to, a Cas9 gene or protein. CRISPR/Cas gene editing systems have been developed to enable targeted modifications to a specific gene of interest in cells. CRISPR/Cas gene editing systems are based on the RNA-guided Cas9 nuclease from the type II prokaryotic clustered regularly interspaced short palindromic repeats (CRISPR) adaptive immune system (see, e.g., Jinek et al., Science, 337: 816 (2012); Gasiunas et al., Proc. Natl. Acad. Sci. U.S.A., 109, E2579 (2012); Garneau et al., Nature, 468: 67 (2010); Deveau et al., Annu. Rev. Microbiol., 64: 475 (2010); Horvath and Barrangou, Science, 327: 167 (2010); Makarova et al., Nat. Rev. Microbiol., 9, 467 (2011); Bhaya et al., Annu. Rev. Genet., 45, 273 (2011); Cong et al., Science, 339: 819-823 (2013); and U.S. Patents 8,697,359, 8,795,965, and 9,322,037; all of which are herein incorporated by reference in their entireties).

In some embodiments, the nucleic acid is a guide RNA (gRNA) that is compatible with CRISPR/Cas gene editing systems, such as gRNA, that comprises CRISPR targeting RNA (crRNA) and trans-activating crRNA (tracrRNA). In some embodiments, crRNA and tracrRNA components of the CRISPR/Cas system are fused into one RNA molecule to target a specific sequence of genomic DNA (gRNAs are not found in nature.). The crRNA sequence is generally synthesized in order to target the desired genomic DNA while the tracrRNA sequence comes from bacterial sequences needed to complex with Cas proteins. Guide RNA can also be referred to as a single guide RNA (sgRNA).

In some embodiments, gRNA and Cas9 protein can comprise a ribonucleoprotein (RNP) complex. RNPs generally include purified Cas9 protein in complex with a gRNA. Such RNPs can be assembled *in vitro* and can be delivered directly to cells using the modified polyamine polymers, such as PEIs and PAMAM dendrimers, of the present disclosure, and in some cases, without the need for electroporation. Cas9 RNPs are capable of cleaving genomic targets with similar efficiency as compared to plasmid-based expression of Cas9/gRNA and can be used for most of the current genome engineering applications of CRISPR, including but not limited to, generating single or multi-gene knockouts in a wide variety of cell types, gene editing using homology directed repair (HDR), and generating large genomic deletions.

In some embodiments, as disclosed further herein, modified polyamine polymers, such as PEIs and PAMAM dendrimers, are used to deliver Cas9 RNPs into a cell to facilitate the insertion of luminescent peptide or polypeptide tag on a protein-of-interest in a cell. In some embodiments, the delivery of such RNPs into cells results in expression of the protein-of-interest and the luminescent tag (e.g., as measured by luminescence of the tag) and/or can result in the production of clones that exhibit stable expression of the protein-of-interest and the luminescent tag. For example, Cas9 RNPs can include Cas9 protein, a gRNA, and a donor DNA template. The donor DNA template can include a polynucleotide encoding a peptide or polypeptide capable of luminescent activity as well as genomic sequences that facilitate insertion of the peptide or polypeptide near an endogenous protein-of-interest (e.g., homology arms). RNPs comprising Cas9 protein, a gRNA, and a donor DNA template can be incorporated into a composition that includes the modified polyamine polymers of the present disclosure to facilitate delivery into a cell for both the transient and stable quantification of protein expression (e.g., luminescence).

In some embodiments, the donor DNA template includes a sequence encoding HiBiT (SEQ ID NO: 3), which is an 11 amino acid polypeptide capable of producing bright and quantitative luminescence through high affinity complementation with an 18 kDa subunit derived from NanoLuc (LgBiT). In accordance with these embodiments, RNPs with donor DNA comprising a sequence encoding HiBiT can be delivered into a cell using the modified polyamine polymers of the present disclosure. In some embodiments, to generate luminescence, a luminogenic substrate and LgBiT (SEQ ID NO: 4) are added to the cell lysates or delivered directly into the cells using the modified polyamine polymers of the present disclosure. Complementation of HiBiT and LgBiT in the presence of the substrate generates a luminescent signal that is proportional to the expression of the protein-of-interest. In some cases, delivery of RNPs and/or luminogenic peptides or polypeptides into cells with the modified polyamine polymers of the present disclosure obviates the need to use other delivery methods, such as electroporation, without causing significant cell toxicity.

In some embodiments, the donor DNA template includes a sequence encoding an 11 amino acid polypeptide (e.g., SmBiT (SEQ ID NO: 5)) capable of producing bright and quantitative luminescence through high affinity complementation with an 18 kDa subunit derived from NanoLuc (e.g., LgBiT). In accordance with these embodiments, RNPs with donor DNA comprising a sequence encoding SmBiT can be delivered into a cell using the modified polyamine polymers of the present disclosure. In some embodiments, to generate luminescence, a luminogenic substrate and LgBiT (SEQ ID NO: 4) are added to the cell lysates or delivered directly into the cells using the modified polyamine polymers of the present disclosure. Complementation of SmBiT and LgBiT in the presence of the substrate generates a luminescent signal that is proportional to the expression of the protein-of-interest. In some cases, delivery of RNPs and/or luminogenic peptides or polypeptides into cells with the modified polyamine polymers of the present disclosure obviates the need to use other delivery methods, such as electroporation, without causing significant cell toxicity.

In some embodiments, a donor DNA template includes a sequence encoding any of the peptides or polypeptides disclosed in U.S. Patent No. 9,797,890, which is incorporated by reference herein in its entirety and for all purposes. For example, the donor template can include a polynucleotide encoding a peptide or polypeptide that comprises a single amino acid difference from MGVTGWRLCERILA (SEQ ID NO: 8). In some embodiments, the donor template can include a polynucleotide encoding a peptide or polypeptide that comprises two or more (e.g., 2, 3, 4, 5, 6, 7, 8, 9, 10, etc.) amino acid differences from MGVTGWRLCERILA (SEQ ID NO: 2), or any other peptides or polypeptides, disclosed in U.S. Patent No. 9,797,890.

In some embodiments, a donor DNA template includes a sequence encoding any of the peptides or polypeptides disclosed in U.S. Provisional Patent Serial No. 62/684,014, which is incorporated by reference herein in its entirety and for all purposes. For example, the donor template can include a polynucleotide encoding a peptide or polypeptide that comprises a single amino acid difference from that of LgTrip 3092 (SEQ ID NO: 9), LgTrip 3546 (SEQ ID NO: 10), LgTrip 2098 (SEQ ID NO: 11), or SmTrip9 (SEQ ID NO: 12). In some embodiments, the donor template can include a polynucleotide encoding a peptide or polypeptide that comprises two or more (e.g., 2, 3, 4, 5, 6, 7, 8, 9, 10, etc.) amino acid differences from SEQ ID NOs: 9-12, or any other peptides or polypeptides, disclosed in U.S. Provisional Patent Serial No. 62/684,014.

Embodiments of the present disclosure also include identifying an optimal modified PEI or PAMAM concentration, or a combination thereof, at optimal concentrations for a biomolecule-of-interest that can provide acceptable results in the context of, for example, transfection efficiency and levels of toxicity. These ratios or concentrations may be determined empirically and will depend on a variety of factors, including but not limited to, the modification percentages of amines in PEIs and PAMAMs, biomolecule-of-interest (e.g., polynucleotide, polypeptide), the types of cells, the cell density, the nature of the assay, and the like. In some embodiments, the optimal concentrations are in the range of nM to µM. In some embodiments, the optimal concentrations are in the range of 1 µM to 20 µM.

Cells that can be used with the compositions and methods of the present disclosure include any suitable prokaryotic or eukaryotic cell. Suitable cells can include those that are easily and reliably grown, have reasonably fast growth rates, have well characterized expression systems, and can be transformed or transfected easily and efficiently. Examples of suitable prokaryotic cells include, but are not limited to, cells from the genera *Bacillus* (such as *Bacillus subtilis* and *Bacillus brevis), Escherichia* (such as *E. coli*), *Pseudomonas, Streptomyces, Salmonella,* and *Erwinia.* Particularly useful prokaryotic cells include the various strains of *Escherichia coli* (e.g., K12, HB101 (ATCC No. 33694), DH5a, DH10, MC1061 (ATCC No. 53338), and CC102). Suitable eukaryotic cells are known in the art and include, for example, yeast cells, insect cells, and mammalian cells, including primary cells and transformed cells. In some embodiments, the cell is a mammalian cell. A number of suitable mammalian host cells are known in the art, and many are available from the American Type Culture Collection (ATCC, Manassas, VA). Examples of suitable mammalian cells include, but are not limited to, Chinese hamster ovary cells (CHO) (ATCC No. CCL61), CHO DHFR- cells (Urlaub et al, Proc. Natl. Acad. Sci. USA, 97: 4216-4220 (1980)), human embryonic kidney (HEK) 293 or 293T cells (ATCC No. CRL1573), and 3T3 cells (ATCC No. CCL92). Other suitable mammalian cell lines are the monkey COS-1 (ATCC No. CRL1650) and COS- 7 cell lines (ATCC No. CRL1651), as well as the CV-1 cell line (ATCC No. CCL70).

Embodiments of the present disclosure also include kits comprising the various components described herein. For example, a kit can include any of the modified polyamine polymer compounds or salts thereof described herein, along with a container and/or instructions. A kit may also include at least one of a DNA molecule, an RNA molecule, a peptide, a polypeptide, a protein, or any combinations or derivatives thereof. In some embodiments, the kit includes a peptide or polypeptide of a Cas9 protein. In some embodiments, the kit includes a donor DNA template comprising a sequence encoding a luminescent peptide or polypeptide (e.g., HiBiT, LgBiT). In some embodiments, the kit includes a gRNA. In some embodiments, the kit includes an RNP complex comprising, for example, a peptide or polypeptide of a Cas9 protein, a donor DNA template comprising a sequence encoding a luminescent peptide or polypeptide (e.g., HiBiT, LgBiT), and a gRNA.

### 4. Examples

It will be readily apparent to those skilled in the art that other suitable modifications and adaptations of the methods of the present disclosure described herein are readily applicable and appreciable, and may be made using suitable equivalents without departing from the scope of the present disclosure or the aspects and embodiments disclosed herein. Having now described the present disclosure in detail, the same will be more clearly understood by reference to the following examples, which are merely intended only to illustrate some aspects and embodiments of the disclosure, and should not be viewed as limiting to the scope of the disclosure. The disclosures of all journal references, U.S. patents, and publications referred to herein are hereby incorporated by reference in their entireties.

The present disclosure has multiple aspects, illustrated by the following non-limiting examples.

In the Examples, the following abbreviations are used: Boc is tert-butyloxycarbonyl; DMEM is Dulbecco's Modified Eagle Medium; DMF is N,N-dimethylformamide; EtOH is ethanol; FBS is fetal bovine serum; MeOH is methanol; THF is tetrahydrofuran;

### Example 1

### Compound Syntheses

### I. Carbamate modifications

### Syntheses of fluorinated alkyl p-nitrobenezene carbonates.

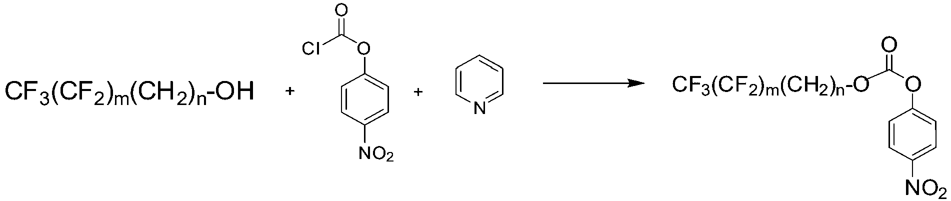

To a solution of fluorinated alcohol (1 eq.) and p-nitrobenzene chloroformate (1.5 eq.) in dry THF, dry pyridine (3 eq.) was added at 0°C. The mixture was stirred at room temperature overnight. After removing the white solid by filtration, the solvent of the filtrate was evaporated, and the residue was purified by silica column using heptane/ethyl acetate as eluent to give the desired compound in yields of 80-90%.

**4-Nitrophenyl (3,3,4,4,5,5,6,6,7,7,8,8,8-tridecafluorooctyl) carbonate (WZ-0845):** ¹H NMR (CD₂Cl₂, δ ppm): 8.31 (d, 2H), 7.46 (d, 2H), 4.62 (t, 2H, CH2O), 2.69 (m, 2H, CH2). MS (m/e) [M+H] (C₁₅H₈F₁₃NO₅): calculated 529.21, observed 529.4.

**4-Nitrophenyl (3,3,4,4,5,5,6,6,6-nonafluorohexyl) carbonate (WZ-0921).** ¹H NMR (CD₂Cl₂, δ ppm): 8.33 (d, 2H), 7.47 (d, 2H), 4.64 (t, 2H, CH2O), 2.70 (m, 2H, CH2). MS (m/e) [M+H] (C₁₃H₈F₉NO₅): calculated 429.19, observed 430.2.

**4-Nitrophenyl (2,2,3,3,4,4,5,5,6,6,6-undecafluorohexyl) carbonate (WZ-0851).** ¹H NMR (CD₂Cl₂, δ ppm): 8.34 (d, 2H), 7.47 (d, 2H), 4.84 (t, 2H, CH2). MS (m/e) [M+H] (C₁₃H₆F₁₁NO₅): calculated 465.18, observed 465.2.

**4-Nitrophenyl (2,2,3,3,4,4,5,5,5-nonafluoropentyl) carbonate (WZ-0886).** ¹H NMR (CD₂Cl₂, δ ppm): 8.34 (d, 2H), 7.47 (d, 2H), 4.85 (t, 2H, CH2). MS (m/e) [M+H] (C₁₂H₆F₉NO₅): calculated 415.17, observed 415.3.

**4-Nitrophenyl (3,3,4,4,5,5,5-heptafluoropentyl) carbonate (WZ-0887).** ¹H NMR (CD₂Cl₂, δ ppm): 8.32 (d, 2H), 7.45 (d, 2H), 4.64 (t, 2H, CH2O), 2.68 (m, 2H, CH2). MS (m/e) [M+H] (C₁₂H₈F₇NO₅): calculated 379.19, observed 379.2.

**Syntheses of carbamate modified PEI 7677.** As shown in Scheme 4, to 4 vials containing a solution of PEI (MW 800) and 150 mg (3.49 mmol monomer) in 5 ml THF/1 ml MeOH 0.1g/ml of 4-nitrophenyl (3,3,4,4,5,5,6,6,6-nonafluorohexyl) carbonate (WZ-0850), THF solution was added with the amounts equivalent to the mole percentages of monomer 16% (0.24g, 0.56 mmol), 27% (0.40g, 0.94 mmol), 42% (0.63g, 1.46 mmol), and 53% (0.79g, 1.84 mmol), respectively. The resulted solutions were stirred over two days. The four individual solutions were then transferred in four Float-A-Lyzer G2 Dialysis Devices (0.5-1.0 kD, 10 ml) and sequentially dialyzed with in MeOH, 0.02M HCl in MeOH, and 0.02M HCl in H₂O over three days. The mixtures were further transferred to 4 tubes with t-butanol (10 ml). The resulted 4 mixtures were filtered into 4 vials, the filtrates lyophilized over two days, and the white powders with 4 different theoretical modification percentages 7667-a-1, 7667-a-2, 7667-a-3, and 7667-a-4 were obtained. The actual modification percentages obtained by ¹H NMR were 10.2%, 16.4%, 22.0%, and 30.0%, respectively.

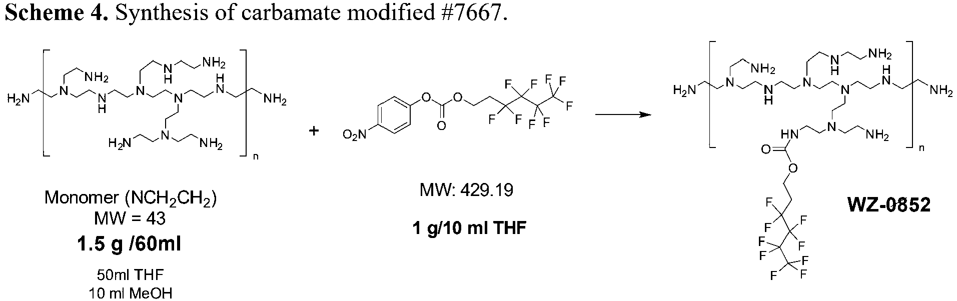

| **#7667** | **PEI MW=800** | **Monomer MW 43** | **4-Nitrobenzene-carbonate** | **Theoretical Modification%** | **Modification NMR%** |
|---|---|---|---|---|---|
| **#7667-a-1** | 150 mg | 3.49 mmol | 0.24 g, 0.56 mmol | 16% | 10.2% |
| **#7667-a-2** | 150 mg | 3.49 mmol | 0.40 g, 0.94 mmol | 27% | 16.4% |
| **#7667-a-3** | 150 mg | 3.49 mmol | 0.63 g, 1.46 mmol | 42% | 22.0% |
| **#7667-a-4** | 150 mg | 3.49 mmol | 0.79 g, 1.84 mmol | 53% | 30.0% |

The other carbamate modified branched PEIs, linear PEIs, or PAMAMs were prepared by employing the similar methods described for 7667. The theoretical and actual percentages of modifications for PEI monomers or PAMAM primary amines were listed in Table 2.

**Table 2. Theoretical and actual modification percentages of PEI monomers for different sizes or PAMAM free amines for different generations modified by different fluorinated carbamates.**

| **Compounds** | **Polymer** | **Modification Tails** | **Theoretical mdf%** | **NMR%** |
|---|---|---|---|---|
| **7668(WZ-0856)-a-1** | **PEI 25000** | **-C(O)O-(CH₂)₂(CF₂)₅CF₃** | **3%** | **2%** |
| a-2 | | | 6% | 4% |
| a-3 | | | 9% | 6% |
| a-4 | | | 12% | 9% |
| a-5 | | | 17% | 12% |
| a-6 | | | 20% | 14% |
| **7675(WZ-0857)-a-1** | **PEI 800** | **-C(O)O-(CH₂)₂(CF₂)₅CF₃** | **9%** | **18%** |
| a-2 | | | 16% | 20% |
| a-3 | | | 25% | 25% |
| a-4 | | | 32% | 25% |
| **7666 (WZ-0853)-a-1** | **PEI 25000** | **-C(O)O-(CH₂)₂(CF₂)₃CF₃** | **3%** | **3%** |
| a-2 | | | 6% | 6% |
| a-3 | | | 9% | 12% |
| a-4 | | | 12% | 16% |
| a-5 | | | 17% | 21% |
| a-6 | | | 20% | 29% |
| **7667 (WZ-0852)-a-1** | **PEI 800** | **-C(O)O-(CH₂)₂(CF₂)₃CF₃** | **16%** | **10%** |
| a-2 | | | 27% | 16% |
| a-3 | | | 42% | 22% |
| a-4 | | | 53% | 30% |
| **7729 (WZ-0882)-a-1** | **PEI 600** | **-C(O)O-(CH₂)₂(CF₂)₃CF₃** | **20%** | **22%** |
| a-2 | | | 30% | 44% |
| a-3 | | | 40% | insoluble in water |
| a-4 | | | 50% | insoluble in water |
| **7730(WZ-0885)-a-1** | **PEI 1200** | **-C(O)O-(CH₂)₂(CF₂)₃CF₃** | **20%** | **24%** |
| a-2 | | | 30% | 44% |
| a-3 | | | 40% | insoluble in water |
| a-4 | | | 50% | insoluble in water |
| **7731 (WZ-0883)-a-1** | **PEI 1800** | **-C(O)O-(CH₂)₂(CF₂)₃CF₃** | **20%** | **21%** |
| a-2 | | | 30% | 35% |
| a-3 | | | 40% | insoluble in water |
| a-4 | | | 50% | insoluble in water |
| **7732 (WZ-0884)-a-1** | **PEI 10000** | **-C(O)O-(CH₂)₂(CF₂)₃CF₃** | **15%** | **20%** |
| a-2 | | | 20% | insoluble in water |
| a-3 | | | 25% | insoluble in water |
| **7738 (wz-0891)-a-1** | **PEI 600** | **-C(O)O-(CH₂)₂(CF₂)₂CF₃** | **20%** | **15%** |
| a-2 | | | 30% | 23% |
| a-3 | | | 40% | 33% |
| a-4 | | | 50% | 41% |
| **7739 (wz-0892)-a-1** | **PEI 800** | **-C(O)O-(CH₂)₂(CF₂)₂CF₃** | **20%** | **21%** |
| a-2 | | | 30% | 29% |
| a-3 | | | 40% | 34% |
| a-4 | | | 50% | 36% |
| **7740 (wz-0893)-a-1** | **PEI 1200** | **-C(O)O-(CH₂)₂(CF₂)₂CF₃** | **20%** | **17%** |
| a-2 | | | 30% | 27% |
| a-3 | | | 40% | 34% |
| a-4 | | | 50% | Partially soluble |
| **7741 (wz-0894)-a-1** | **PEI 1800** | **-C(O)O-(CH₂)₂(CF₂)₂CF₃** | **20%** | **17%** |
| a-2 | | | 30% | 26% |
| a-3 | | | 40% | 34% |
| a-4 | | | 50% | Partially soluble |
| **7782 (wz-0923)-a-1** | **PEI 10000** | **-C(O)O-(CH₂)₂(CF₂)₂CF₃** | **8%** | |
| a-2 | | | 12% | |
| a-3 | | | 16% | |
| a-4 | | | 20% | |
| a-5 | | | 24% | |
| **7783 (wz-0924)-a-1** | **PEI 25000** | **-C(O)O-(CH₂)₂(CF₂)₂CF₃** | **8%** | |
| a-2 | | | 12% | |
| a-3 | | | 16% | |
| a-4 | | | 20% | |
| a-5 | | | 24% | |
| **7742 (wz-0895)-a-1** | **PEI 600** | **-C(O)O-(CH₂)₂CF₂CF₃** | **20%** | **Membrane leak** |
| a-2 | | | 30% | 21% |
| a-3 | | | 40% | 29% |
| a-4 | | | 50% | 35% |
| **7743 (wz-0896)-a-1** | **PEI 800** | **-C(O)O-(CH₂)₂CF₂CF₃** | **20%** | **22%** |
| a-2 | | | 30% | 30% |
| a-3 | | | 40% | 37% |
| a-4 | | | 50% | 44% |
| **7744 (wz-0897)-a-1** | **PEI 1200** | **-C(O)O-(CH₂)₂CF₂CF₃** | **20%** | **19%** |
| a-2 | | | 30% | 24% |
| a-3 | | | 40% | 32% |
| a-4 | | | 50% | 39% |
| **7745 (wz-0898)-a-1** | **PEI 1800** | **-C(O)O-(CH₂)₂CF₂CF₃** | **20%** | **18%** |
| a-2 | | | 30% | 22% |
| a-3 | | | 40% | 29% |
| a-4 | | | 50% | 39% |
| **7758 (wz-0902)-a-1** | **PEI 600** | **-C(O)O-CH₂(CF₂)₂CF₃** | **20%** | **NMR cannot differentiate** |
| a-2 | | | 25% | |
| a-3 | | | 30% | |
| **7759 (wz-0903)-a-1** | **PEI 800** | **-C(O)O-CH₂(CF₂)₂CF₃** | **20%** | **NMR cannot differentiate** |
| a-2 | | | 25% | |
| a-3 | | | 30% | |
| **7760 (wz-0904)-a-1** | **PEI 1200** | **-C(O)O-CH₂(CF₂)₂CF₃** | **20%** | **NMR cannot differentiate** |
| a-2 | | | 25% | |
| a-3 | | | 30% | |
| **7766(WZ-0908)-a-1** | **PAMAM G1** | **-C(O)O-(CH₂)₂(CF₂)₂CF₃** | **20%** | |
| a-2 | | | 30% | |
| a-3 | | | 40% | |
| a-4 | | | 50% | |
| **7767(WZ-0909)-a-1** | **PAMAM G3** | **-C(O)O-(CH₂)₂(CF₂)₂CF₃** | **20%** | |
| a-2 | | | 30% | |
| a-3 | | | 40% | |
| a-4 | | | 50% | |
| **7768(WZ-0910)-a-1** | **PAMAM G5** | **-C(O)O-(CH₂)₂(CF₂)₂CF₃** | **20%** | |
| a-2 | | | ^{~}100% | |
| a-3 | | | ^{~}100% | |
| a-4 | | | ^{~}100% | |
| b-1 | | | 40% | |
| b-2 | | | 50% | |
| **7825(WZ-0949)-a-1** | **PAMAM G2** | **-C(O)O-(CH₂)₂(CF₂)₂CF₃** | **30%** | |
| a-2 | | | 40% | |
| a-3 | | | 50% | |
| **7826(WZ-0950)-a-1** | **PAMAM G7** | **-C(O)O-(CH₂)₂(CF₂)₂CF₃** | **30%** | |
| a-2 | | | 40% | |
| a-3 | | | 50% | |
| **7829(WZ-0952)-a-1** | **PAMAM G4** | **-C(O)O-(CH₂)₂(CF₂)₂CF₃** | **30%** | |
| a-2 | | | 40% | |
| a-3 | | | 50% | |
| **7830(WZ-0953)-a-1** | **PAMAM G6** | **-C(O)O-(CH₂)₂(CF₂)₂CF₃** | **30%** | |
| a-2 | | | 40% | |
| a-3 | | | 50% | |
| **7838(WZ-0957)-a-1** | **Linear PEI-10K** | **-C(O)O-(CH₂)₂(CF₂)₃CF₃** | **18%** | **18%** |
| a-2 | | | 26% | 29.00% |
| a-3 | | | 35% | 40% |
| a-4 | | | 44% | 50% |
| **7839(WZ-0958)-a-1** | **Linear PEI-10K** | **-C(O)O-(CH₂)₂(CF₂)₂CF₃** | **20%** | **22%** |
| a-2 | | | 30% | 33% |
| a-3 | | | 40% | 43% |
| a-4 | | | 50% | 59% |
| **7840(WZ-0961)-a-1** | **Linear PEI-25K** | **-C(O)O-(CH₂)₂(CF₂)₃CF₃** | **18%** | **23%** |
| a-2 | | | 26% | 39% |
| a-3 | | | 35% | 34% |
| a-4 | | | 44% | Partially soluble in water |
| **7841(WZ-0962)-a-1** | **Linear PEI-25K** | **-C(O)O-(CH₂)₂(CF₂)₂CF₃** | **20%** | **27%** |
| a-2 | | | 30% | 48% |
| a-3 | | | 40% | 57% |
| a-4 | | | 50% | Partially soluble in water |
| **7842(WZ-0963)-a-1** | **Linear PEI-2.5K** | **-C(O)O-(CH₂)₂(CF₂)₃CF₃** | **20%** | **37%** |
| a-2 | | | 30% | 49% |
| a-3 | | | 40% | Not soluble in water |
| **7843(WZ-0964)-a-1** | **Linear PEI-250K** | **-C(O)O-(CH₂)₂(CF₂)₃CF₃** | **20%** | **38%** |
| a-2 | | | 30% | Not soluble in water |
| a-3 | | | 40% | Not soluble in water |

### II. Imine reduction modifications

**Syntheses of PEI 7636 modified by imine reduction.** As shown in Scheme 5, to 4 vials containing the solution of PEI (MW 800) 100 mg (2.33 mmol monomer) in 2 ml MeOH, a solution of 3,3,4,4,5,5,6,6,7,7,8,8,8-tridecafluorooctanal (0.5g/ml) in THF was added with the amounts equivalent to the mole percentages of monomer 16% (0.135g, 0.37 mmol), 21% (0.181g, 0.50 mmol), 27% (0.226g, 0.62 mmol), and 38% (0.315g, 0.87 mmol), respectively. The resulted solutions were stirred overnight. To the above 4 corresponding solutions, NaBH₄ 43 mg, 57 mg, 71 mg, 85 mg and 99 mg, respectively, were slowly added at 0°C. The mixtures were stirred at room temperature for 2 hours till the bubbles disappeared. The four individual solutions were then transferred into four Float-A-Lyzer G2 Dialysis Devices (0.5-1.0 kD, 10 ml) and sequentially dialyzed with in MeOH, 0.02M HCl in MeOH, and 0.02M HCl in H₂O over three days. The mixtures were transferred to 4 different tubes with t-butanol (10 ml). The resulted 4 mixtures were filtered into 4 vials, the filtrates were lyophilized over two days, and the pale yellow powders with 4 different theoretical modification percentages 7636-b-1, 7636-b-2, 7636-b-3, and 7636-b-4 were obtained.

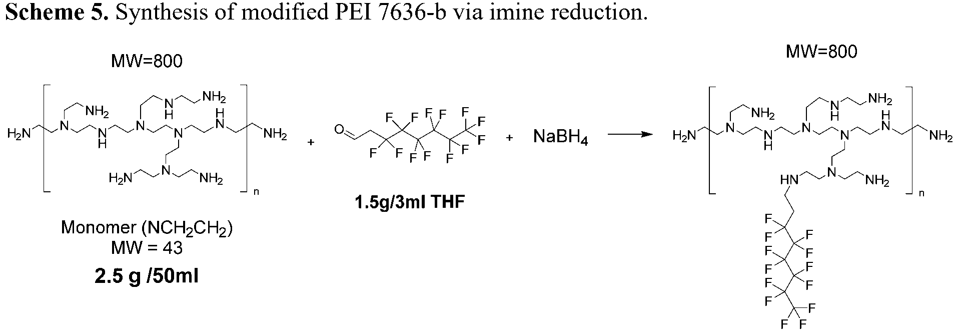

| **Compounds** | **PEI MW 800** | **Monomer MW 43** | **3,3,4,4,5,5,6,6,7,7,8,8,8 -tridecafluorooctanal MW 362.09** | **NaBH₄ MW 37.83** | **Theoretical %** |
|---|---|---|---|---|---|
| **7636-b-1** | 100 mg | 2.33 mmol | 0.135 g, 0.37mmol | 43 mg | 16% |
| **7636-b-2** | 100 mg | 2.33 mmol | 0.181 g, 0.50mmol | 57 mg | 21% |
| **7636-b-3** | 100 mg | 2.33 mmol | 0.226 g, 0.62 mmol | 71 mg | 27% |
| **7636-b-4** | 100 mg | 2.33 mmol | 0.271 g, 0.75 mmol | 85 mg | 32% |
| **7636-b-5** | 100 mg | 2.33 mmol | 0.315 g, 0.87 mmol | 99 mg | 38% |

The other imine-reduction modified branched PEIs were prepared by employing the similar methods described for 7636. The theoretical percentages of modifications for PEI monomers are listed in Table 3.

**Table 3. Theoretical modification percentages of PEI monomers of different sizes modified with different fluorinated aldehyde by imine reduction.**

| **Compounds** | **Polymer** | | **Modified Tails** | **Theoretical %** |
|---|---|---|---|---|
| **7636(WZ-0867)-b-1** | **PEI 800** | **Imine reduction** | **-(CH₂)₂(CF₂)₅CF₃** | **16%** |
| b-2 | | | | 21% |
| b-3 | | | | 27% |
| b-4 | | | | 32% |
| b-5 | | | | 38% |
| **7637(WZ-0868)-b-1** | **PEI 25000** | **Imine reduction** | **-(CH₂)₂(CF₂)₅CF₃** | **4%** |
| b-2 | | | | 6% |
| b-3 | | | | 8% |
| b-4 | | | | 10% |
| b-5 | | | | 12% |
| **7709(WZ-0869)-a-1** | **PEI 800** | **Imine reduction** | **-(CH₂)₂(CF₂)₇CF₃** | **16%** |
| a-2 | | | | 21% |
| a-3 | | | | 27% |
| a-4 | | | | 32% |
| a-5 | | | | 38% |
| **7710(WZ-0870)-a-1** | **PEI 25000** | **Imine reduction** | **-(CH₂)₂(CF₂)₇CF₃** | **4%** |
| a-2 | | | | 6% |
| a-3 | | | | 8% |
| a-4 | | | | 10% |
| a-5 | | | | 12% |

Pentafluorobenzyl modified PEIs 7669 and 7677 and pyridine methyl modified PEI 7676 can be prepared *via* imine reduction by employing the similar method. The theoretical and actual modification percentages of PEI monomers were listed in Table 4.

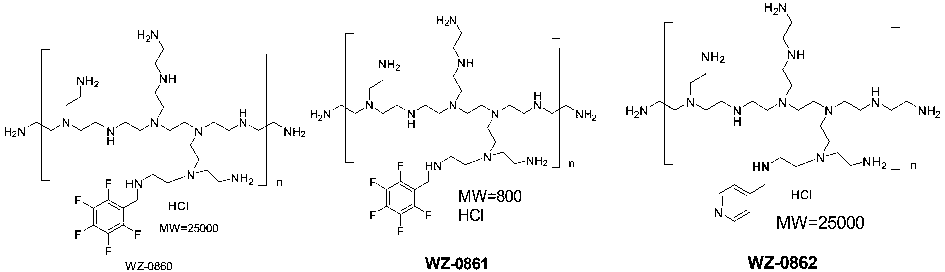

**Table 4. Aromatic ring modified PEIs via imine reduction**

| **Compound** | **PEI** | **Modification Moiety** | **Theoretical%** | **NMR%** |
|---|---|---|---|---|
| **7669(WZ-0860)a-1** | **PEI-25K** | **Pentafluorobenzyl** | **3%** | **1%** |
| a-2 | | | 6% | 3% |
| a-3 | | | 9% | 4% |
| a-4 | | | 12% | 9% |
| **7677 (WZ-0861)-a-1** | **PEI-800** | **Pentafluorobenzyl** | **16%** | **7%** |
| a-2 | | | 27% | 9% |
| a-3 | | | 42% | 13% |
| a-4 | | | 53% | 21% |
| **7676 (WZ-0862)-a-1** | **PEI-25K** | **PyCH2** | **3%** | **0.20%** |
| a-2 | | | 6% | 0.30% |
| a-3 | | | 9% | 0.40% |
| a-4 | | | 12% | 1.70% |
| a-5 | | | 25% | 1.40% |

### III. Alkylated PEI via BOC-protection, alkylation and deprotection.

**BOC-protected PEIs**. 2.5 g (58.1 mmol monomer) of PEI 1200 or PEI 25000 and NaHCO₃ were dissolved in 20 ml THF/40 water. To the solution BOC-anhydride (5.07g, 23.3 mmol) in 20 ml, THF was added at 0°C. The mixture was stirred overnight at room temperature. The mixture was then heated to 65°C overnight. TLC confirmed BOC-anhydride was completely disappeared by iodine staining. After removing THF, the solution was frozen and dried by lyophilization. BOC-protected PEIs were confirmed by ¹H NMR.

**Alkylation of BOC-protected PEIs**. BOC-PEI (0.20 g, 4.65 mmol) was suspended in EtOH or DMF. To the mixture, 1,1,1,2,2,3,3,4,4-nonafluoro-6-iodohexane was added with the amounts equivalent to 30%, 40%, and 50% of PEI monomers, and the resulted mixture was heated to 95°C for 48 hours. After removing EtOH, the residue for each reaction was triturated with ether, or ether was poured into each individual reaction DMF solution, the solvent of was removed by centrifuge. The pale yellow solid from each reaction was then washed with ether and removed by centrifuge two more times and dried under vacuum. The alkylated BOC-PEIs were confirmed by FNMR.

**Deprotection of Alkylated BOC-PEIs.** 12 ml of TFA/DCM(1:1) was added to the above each white solid in the presence of TIPS (0.1 ml). The mixtures were stirred at room temperature for 3 hours. After removing the solvent, the residual solids were triturated with ether and centrifuge three times and dried under vacuum. The solids were dissolved in water and dialyzed in H₂O three times for three days. The solutions were lyophilized, and the pale yellow powders were obtained. The modification percentages were calculated by HNMR and FNMR using CF₃CH₂OH as an internal control (Table 5).

**Table 5. Alkylation percentages of PEI monomers of different sizes via BOC-protection and deprotection**

| **Compounds** | **PEIs** | **alkylation: - ICH₂(CF₂)₃CF₃** | **Theoretical%** | **NMR%** | **Alkylation solvent** |
|---|---|---|---|---|---|
| **7827-(WZ-0954)a-1** | **PEI-1200** | **-CH₂(CF₂)₃CF₃** | **30%** | **3.90%** | EtOH |
| a-2 | | | 40% | 3.70% | EtOH |
| a-3 | | | 50% | 9.70% | EtOH |
| b-1 | | | 40% | 15.60% | DMF |
| b-2 | | | 50% | 17.40% | DMF |
| **7833 (WZ-0955)-a-1** | **PEI-25000** | **-CH₂(CF₂)₃CF₃** | **60%** | **12.10%** | DMF |
| a-2 | | | 40% | 10% | DMF |
| a-3 | | | 50% | 17.80% | DMF |

### Example 2

### Gene Delivery by Modified PEI Polymers

HEK293 cells were plated at 1.5x10⁵ cell/ml in 100µl DMEM/FBS the day before transfecting 10ng/well TK/NanoLuc^{®} expression construct (Promega catalog #N1501) diluted with 90ng/well carrier DNA (Promega catalog #E4881). Cells were transfected with various modified PEI polymers at different concentrations and compared to a FuGENE HD positive control (Promega catalog #E2311) at the standard 3:1 ratio and a negative control of buffer alone. These conditions gave a wide dynamic range for measuring transfection efficiency by addition of Nano-Glo^{®} Luciferase Assay Reagent (Promega catalog #N1110) 24 hours after transfection. In some cases, transfections were also carried out in media lacking serum, which was added back several hours after transfection. Toxicity was measured by adding reconstituted CellTiter-Glo^{®} Reagent (Promega catalog #G7570) to replicate wells.

A 9-to-1 ratio of PEI monomer (N atoms) to DNA phosphorus atoms was initially chosen as the default 1x concentration, rather than using a weight-to-weight ratio. Both transfection efficiency and toxicity are affected by this ratio. The different concentrations of PEIs used relative to that standard 1x molar concentration are reported.

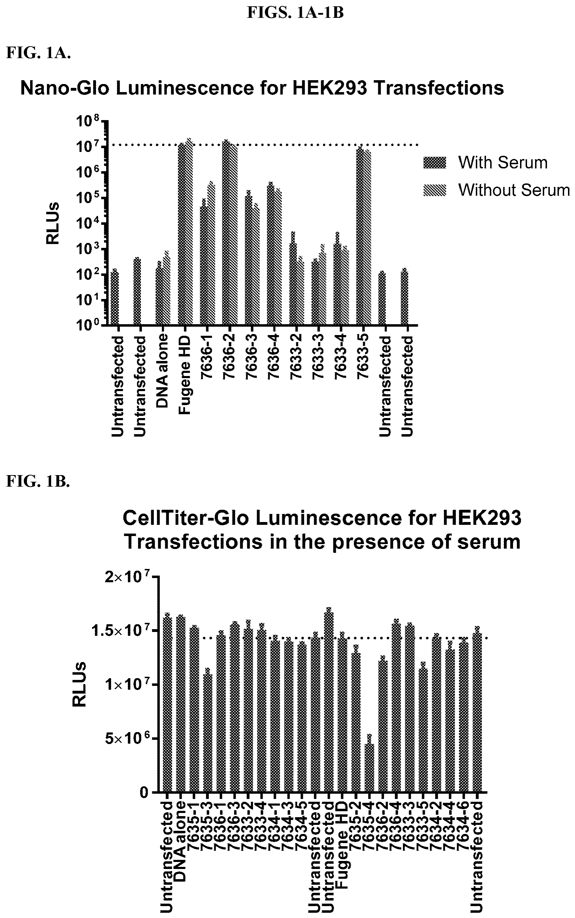

An initial experiment with this fixed ratio of monomer to DNA indicated that modified PEI polymers could be just as effective as FuGENE HD at transfecting HEK293 cells. Transfection efficiency as measured by NanoLuc^{®} expression could be as high as FuGENE HD for some of the compounds, while there was no uptake of DNA in the absence of reagent, as shown in FIG. 1A. The extent of modification of the base polymer had a significant effect on both the transfection efficiency and the toxicity, highlighting the importance of fine-tuning the chemical synthesis for this application. At least at this constant ratio of PEI monomers, the compounds based on the 800 Da polymer were more effective than those based on the 25,000 Da polymer, with 7636-2 and 7633-5 performing particularly well. PEI compounds based on the 25,000 Da polymer generally appeared more sensitive to the presence of serum during transfection showing lower relative transfection, while the 800 Da polymers displayed less of this effect.

Cell viability data are shown in FIG. 1B. Most compounds were well tolerated.

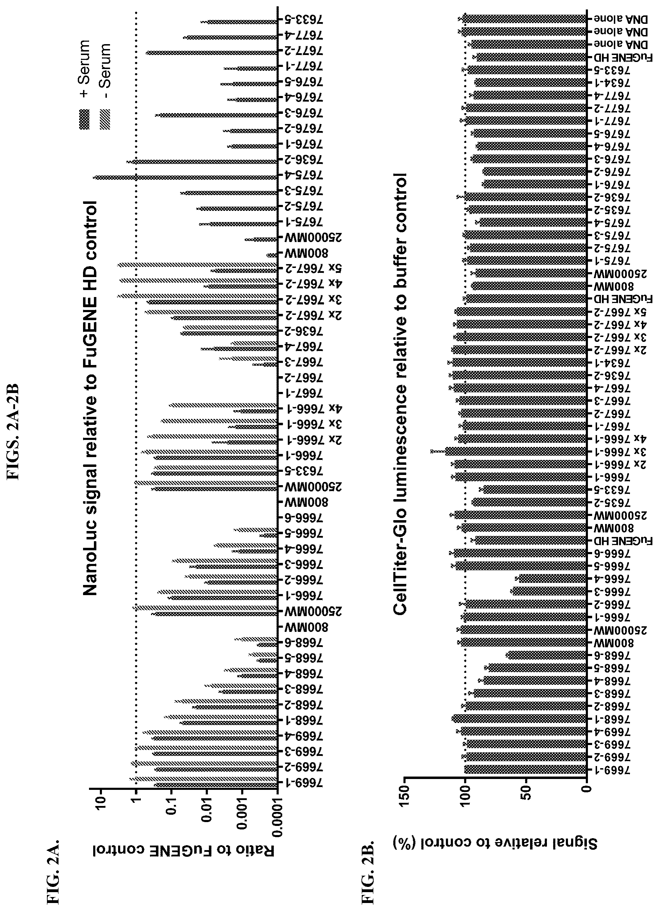

Further experiments demonstrated that additional modifying groups could be used to improve efficiency of transfection or decrease toxicity, especially in comparison to the MW 800Da polymer starting material, which displayed negligible transfection and high toxicity. As seen in FIG. 2, the 25,000Da polymer with no modification already shows relatively high transfection efficiency and low toxicity. The 7669 series of modified 25,000 Da polymer likewise was highly effective at all modification levels. The 7666 series, on the other hand, showed reduced efficiency with increased levels of modification. In the case of 7666-1, increasing the ratio of polymer to DNA decreases transfection efficiency. However, with 7667-2, based on the 800Da polymer, efficient transfection was only seen at concentrations at least double the standard 1x conditions. 7636-2, 7676-3, and 7677-2 all gave good results at the 1x concentration.

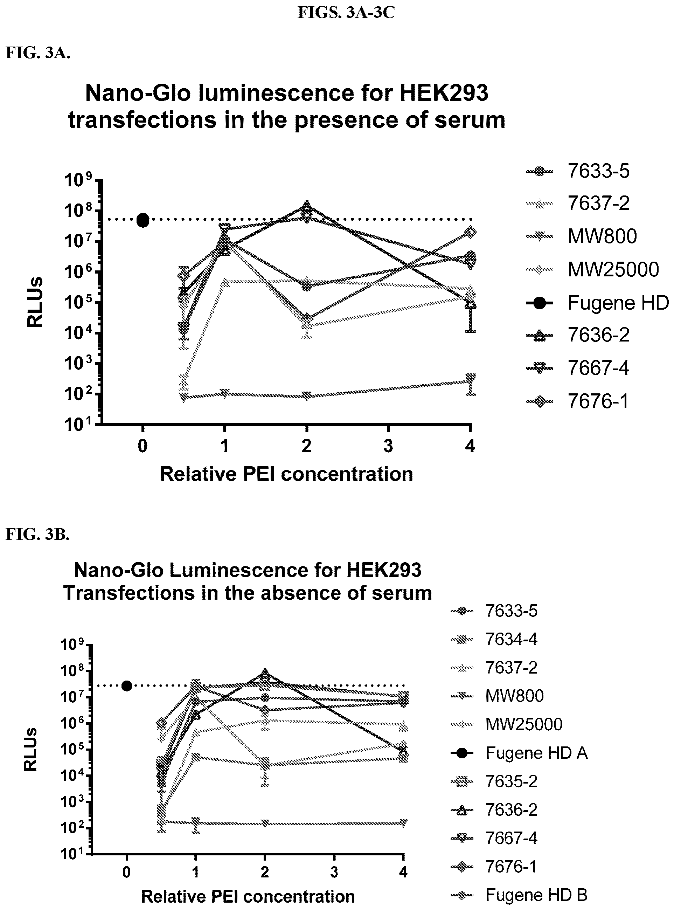

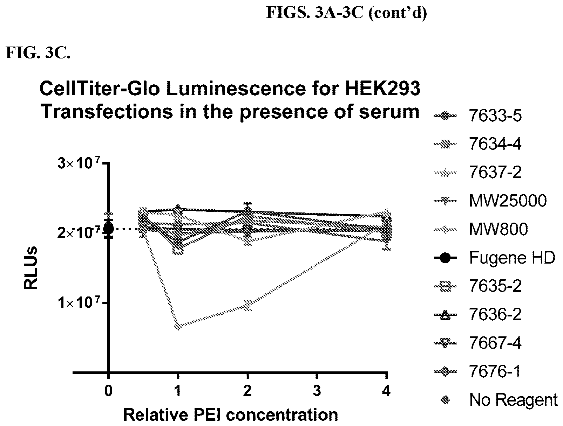

To better determine the optimal concentrations of the various compounds, titrations of various modified PEIs were added to a constant amount of DNA and used to transfect HEK293 cells. Data are shown in FIG. 3, with FIG. 3A showing data in the presence of serum and FIG. 3B showing data in the absence of serum. FIG. 3C shows cell viability as measured using the CellTiter-Glo^{®} Luminescent Cell Viability Assay (Promega catalog #G7570) in the presence of serum. In the legends, MW800 and MW25000 refer to the unmodified PEI polymers of the indicated molecular weights. In this figure, a relative PEI concentration of 1 indicates the default 9-to-1 ratio of PEI monomer to DNA phosphorus. This experiment identified an optimal molar ratio of PEI monomer to DNA for each compound when comparing transfection efficiency. At twice the standard ratio, both 7636-2 and 7667-4 displayed higher transfection efficiency than FuGENE HD without significant toxicity. In the absence of serum, 7676-1 was also able to match FuGENE HD.

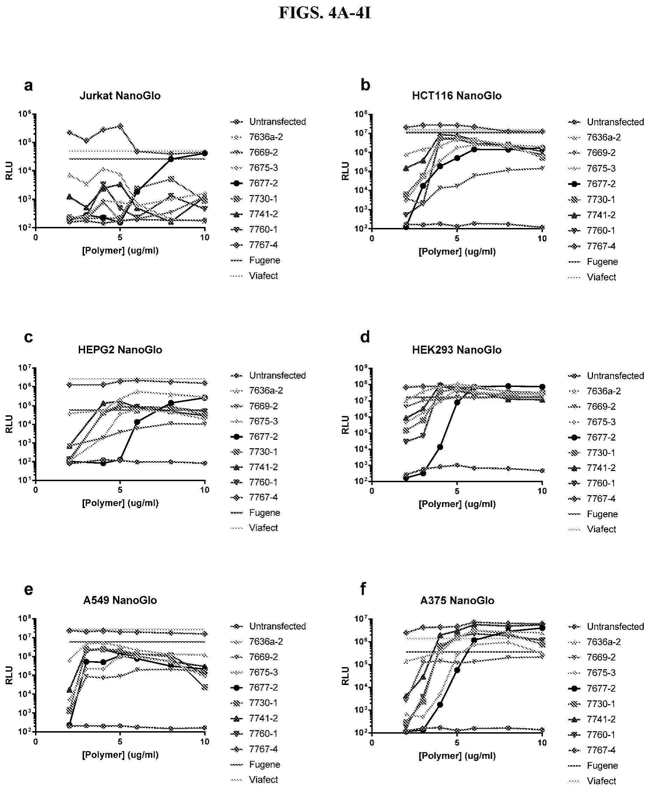

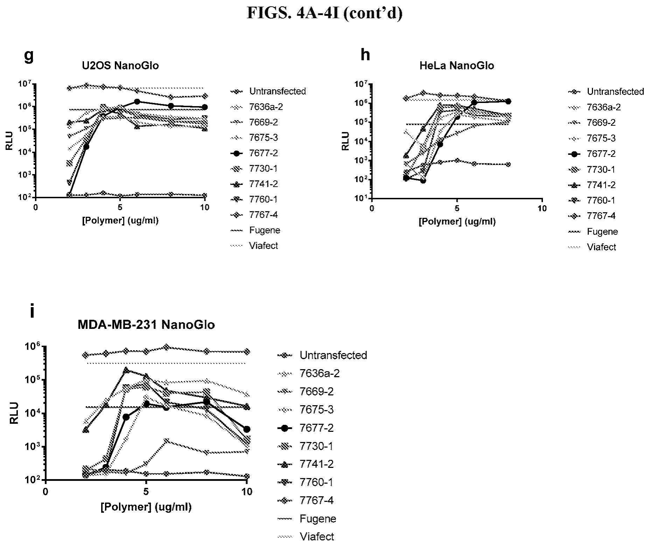

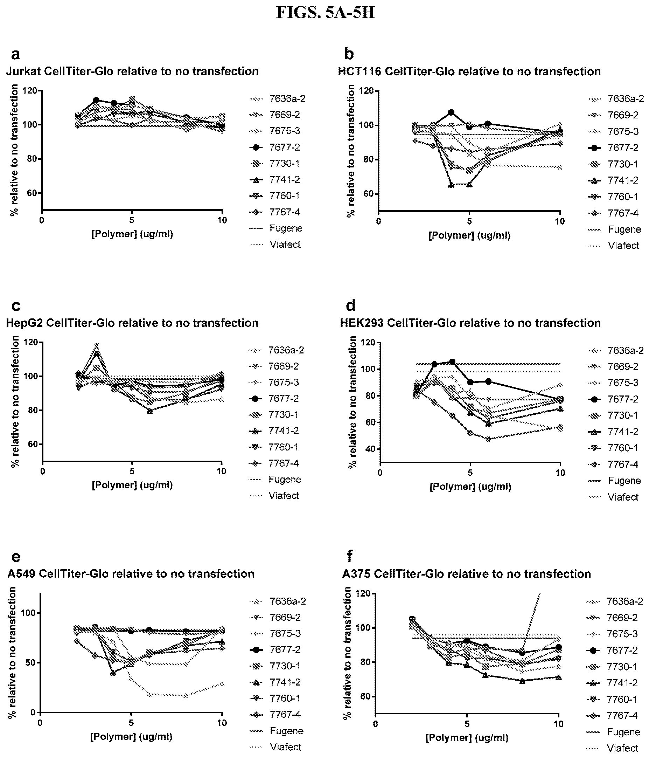

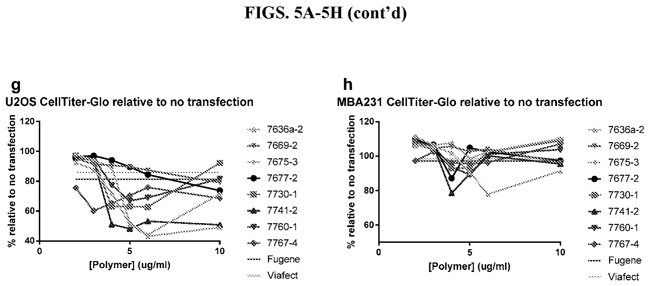

A panel of polymers, including PEI polymers of different molecular weights or PAMAM polymers, were used to transfect nine cell lines at different polymer concentrations. HEK293, HeLa, MDA-MB-231, HepG2, A375, HCT116, U2-OS, Jurkat, and A549 cells were plated at 1.0 x10⁵ cells/ml in 100µl DMEM/FBS (HEK293, HeLa, MDA-MB-231, HepG2, A375, HCT116, U2-OS), RPMI/FBS (Jurkat) or F12/FBS (A549) the day before transfecting 10ng/well TK/NanoLuc^{®} expression construct (Promega catalog #N1501) diluted with 90ng/well carrier DNA (Promega catalog #E4881). Cells were transfected with various modified PEI or PAMAM polymers at different concentrations and compared to FuGENE HD and ViaFect^{™} positive controls (Promega catalog #E2311 and #E4981) at a 3:1 ratio and a negative control of buffer alone. These conditions gave a wide dynamic range for measuring transfection efficiency by addition of Nano-Glo^{®} Luciferase Assay Reagent (Promega catalog #N1110) 24 hours after transfection (FIGS. 4A-4I). Toxicity was measured by adding reconstituted CellTiter-Glo^{®} Reagent (Promega catalog #G7570) to replicate wells (FIGS. 5A-5H). At optimal concentrations, the Generation 3 PAMAM polymer, 7767-4, surpassed or virtually matched the performance of the ViaFect^{™} and FuGENE HD controls for NanoLuc^{®} expression for all cell lines.

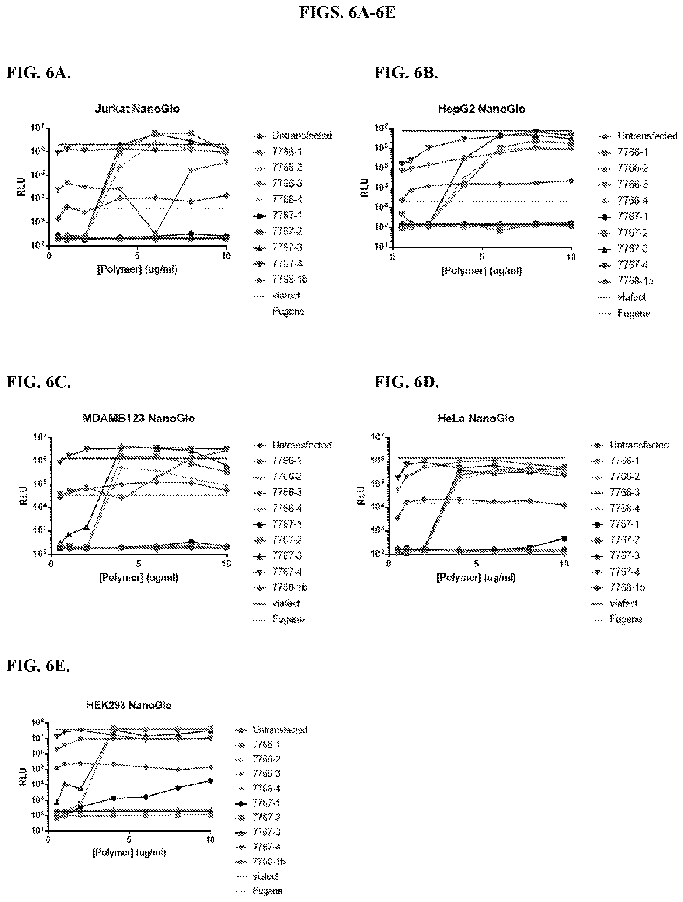

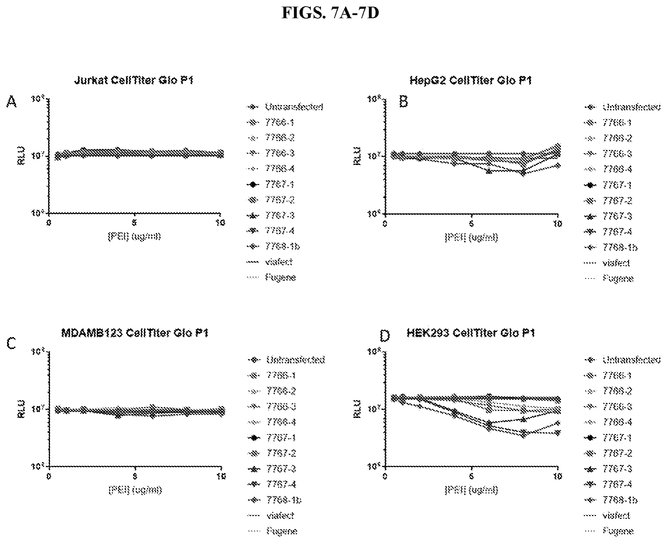

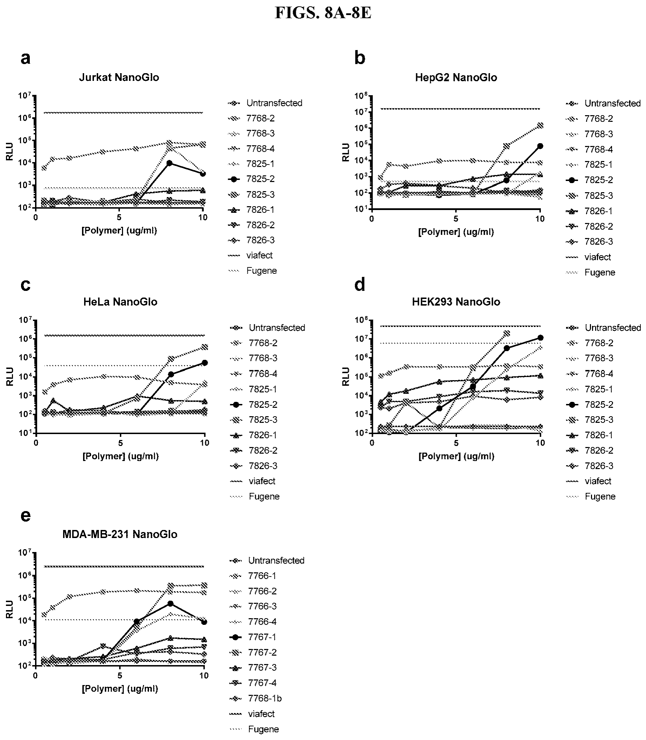

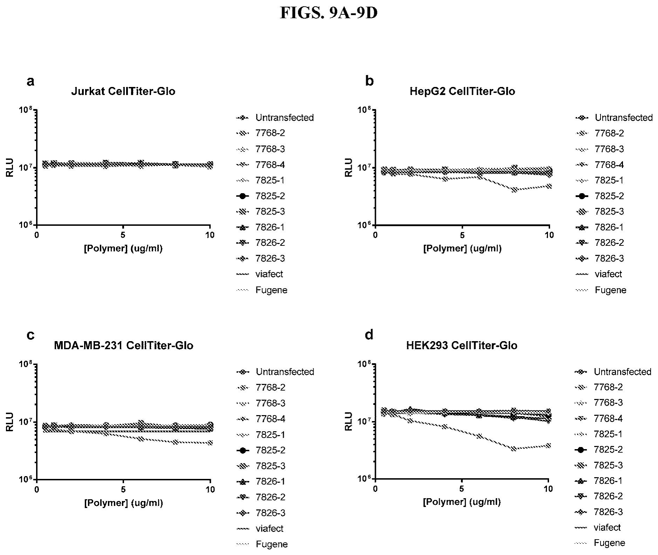

To further investigate the ability of PAMAM polymers to transfect a wide variety of cell types, titrations of PAMAM polymers of different sizes and modification levels were used to transfect five different cell lines (FIGS. 6-9). HEK293, HeLa, MDA-MB-231, HepG2, and Jurkat cells were plated at 1.5 x10⁵ cell/ml in 100µl DMEM/FBS (all cell types except Jurkat which were plated in RPMI/FBS) the day before transfecting 10ng/well TK/NanoLuc^{®} expression construct (Promega catalog #N1501) diluted with 90ng/well carrier DNA (Promega catalog #E4881). Cells were transfected with titrations of various PAMAM polymers: 7766 (-1 to -4), 7767 (-1 to -4) and 7768-1b (FIGS. 6 and 7) and 7768 (-2 to -4), 7825 (-1 to -4) and 7826 (-1 to -4) (FIGS. 8 and 9). FuGENE HD and ViaFect^{™} (Promega catalog #E2311 and #E4981) were used as positive controls at a 3:1 ratio, and buffer alone was used as a negative control. Transfection efficiency was measured using the Nano-Glo^{®} Luciferase Assay (Promega catalog #N1110) 24 hours after transfection (FIGS. 6A-6E and 8A-8E). Toxicity was measured by adding reconstituted CellTiter-Glo^{®} Reagent (Promega catalog #G7570) to replicate wells of all cells except HeLa (FIGS. 7A-7D and 9A-9D). The extent of modification of the polymer had an effect on the optimal concentration and the transfection efficiency. While 7767-4 once again showed effective transfection of all the cell types across a wide range of concentrations, the less-modified versions, 7767-2 and 7767-3, required higher concentrations but could deliver similar, or even greater, amounts of DNA than 7767-4 at those higher concentrations.

### Example 3

### LgBiT Delivery

This example involved CRISPR-mediated tagging as described generally in Schwinn et al. "CRISPR-Mediated Tagging of Endogenous Proteins with a Luminescent Peptide," ACS Chem. Biol. 2018, 13, 467-474. HiBiT-edited cells were generated by CRISPR, and clones were isolated by single cell sorting. The clones were plated at 10,000 cells per well in a 96-well plate. After 24 h, cells were transfected with different complexes of modified PEIs (1 µg/ml) with LgBiT protein (170 nM). The complexes were prepared by incubating modified PEIs (100 µg/ml) with LgBiT (17 µM) in Opti-MEM I reduced serum media for 30 min at room temperature. Cells treated with 10% BacMam-LgBiT to transduce a CMV/LgBiT expression construct served as benchmark control. After 24 h, cells were treated with DarkBiT (SEQ ID NO: 6 or SEQ ID NO: 7), a peptide with high affinity for LgBiT (the LgBiT/DarkBiT complex produces little or no luminescence) (1 µM) to quench luminescence activity of extracellular LgBiT. After 1h, cells were assayed using the Nano-Glo^{®} Live Cell Assay System (Promega catalog #N2011) and CellTiter-Glo^{®} 2.0 Assay (Promega catalog #G7570). Luminescence was measured on a GloMax^{®} Discover. Data were analyzed with Prism 5.0 software (GraphPad). Results are shown in FIGS. 10-11.

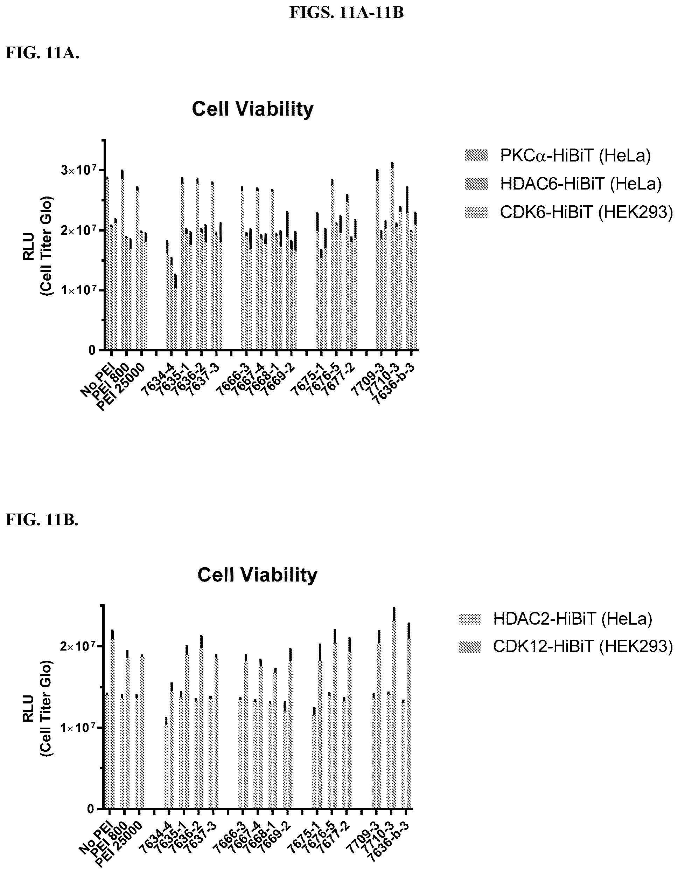

The brightness of the luminescence signal is proportional to the amount of intracellular LgBiT being delivered, which can be correlated to the efficiency of protein uptake. Generally, large size PEIs (MW=25,000), with or without modifications, performed better at LgBiT delivery than their respective small size PEIs (MW=800). Modified large size PEIs were better than the unmodified PEI. Among the top hits, 7666-3, 7668-1, 7669-2, 7675-1, and 7709-3 worked best at facilitating LgBiT uptake across 5 different HiBiT clones (FIGS. 10 and 11). Of the 6 best candidates, 7669-2 and 7675-1 caused marginal cytotoxicity. No toxicity was observed with 7666-3, 7668-1, 7709-3 (FIGS. 11A and 11B).

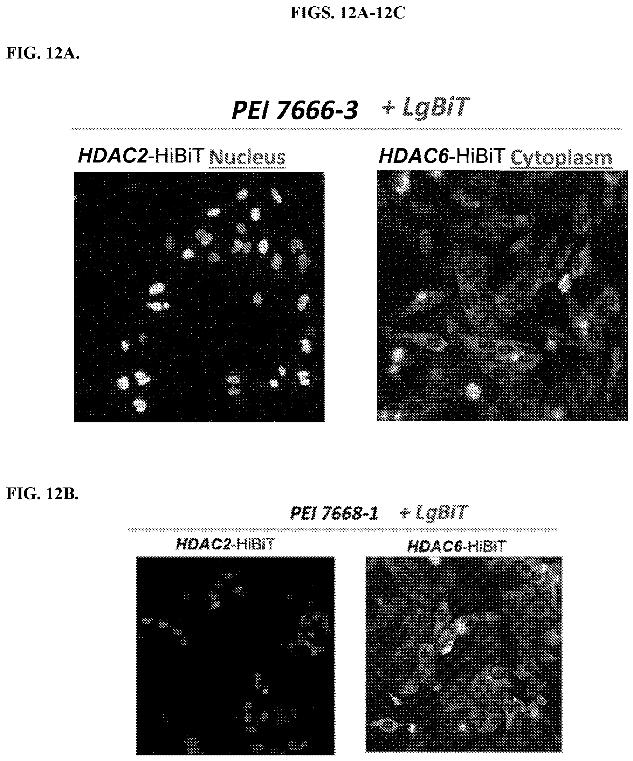

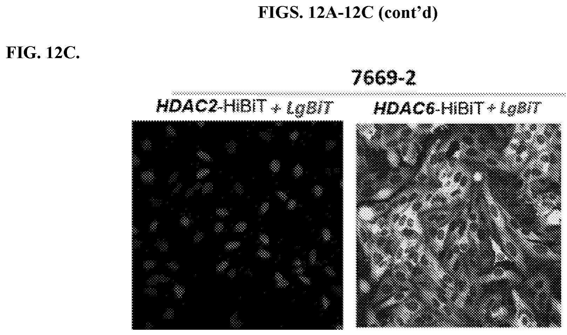

HDAC2-HiBiT and HDAC6-HiBiT clones of HeLa cells were plated at 25,000 cells per well in an 8-well chamber slide. After 24 h, cells were transfected with different complexes of PEI (1 µg/ml) and LgBiT protein (170 nM). The complexes were prepared by incubating 17 µM LgBiT with 100 µg/ml 7666-4, 7668-1, or 7669-2 in Opti-MEM I reduced serum media for 30 min at room temperature. After 24 h, cells were switched to CO₂ independent media, and Nano-Glo^{®} Live Cell substrate was added. Cells were imaged with a bioluminescence imager (FIGS. 12A-12C).

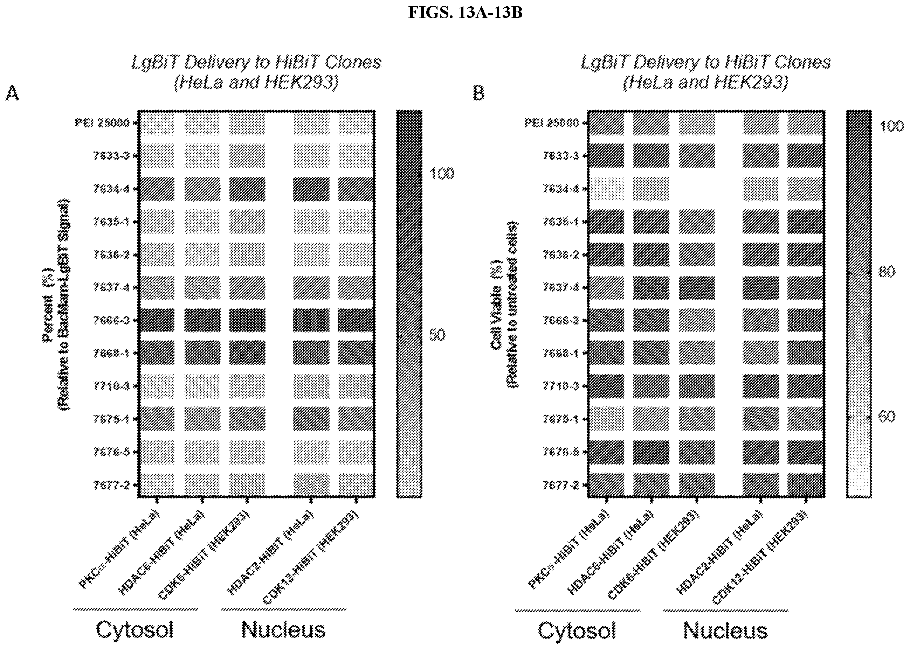

HiBiT-edited HeLa or HEK293 cells were plated at 10,000 cells/well in wells of a 96-well plate. After 24hrs, cells were transfected with different complexes of PEIs (modified and unmodified; 5ug/ml) and LgBiT protein (200nM). The complexes were prepared by incubating the PEI with LgBiT in Opti-MEM reduced serum media for 30 minutes at room temperature. Cells treated with 10% BacMam-LgBiT to transduce a CMV-LgBiT expression construct served as a control. After 24hrs, cell medium was replaced, and cells assayed using NanoGlo^{®} Live Cell Assay System (Promega Cat. # N2011) and CellTiter-Glo^{®} 2.0 Assay (Promega Cat. No. G7570). Luminescence was measure on a GloMax^{®} Discover. FIG. 13A depicts the percent of LgBiT protein delivered in comparison to LgBiT protein delivered via BacMam transduction. The brightness of the luminescence signal is proportional to the amount of intracellular LgBiT being delivered to the cells, which can be correlated to the efficiency of protein uptake. FIG. 13B depicts the percentage of live cells relative to untreated cells.

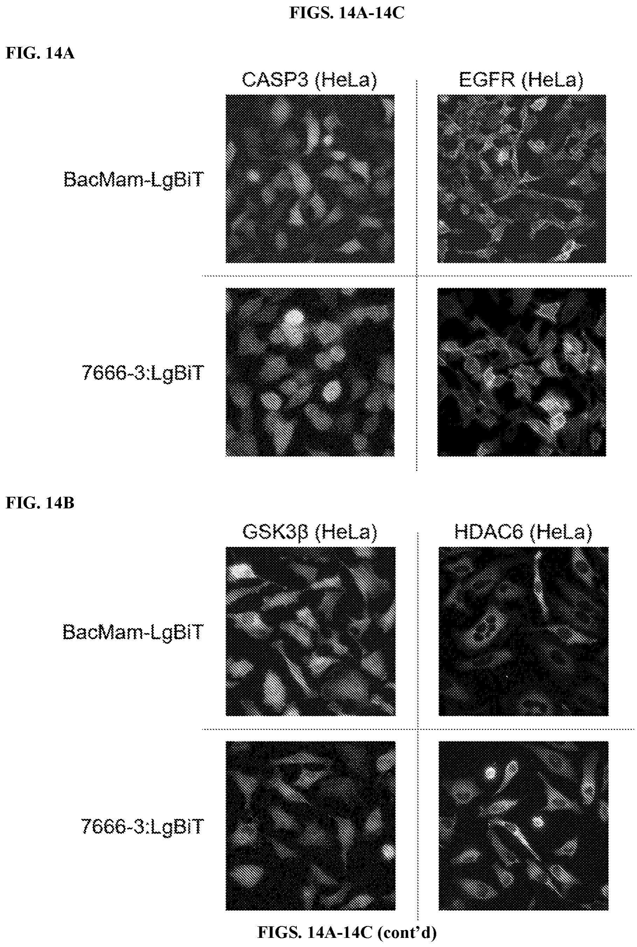

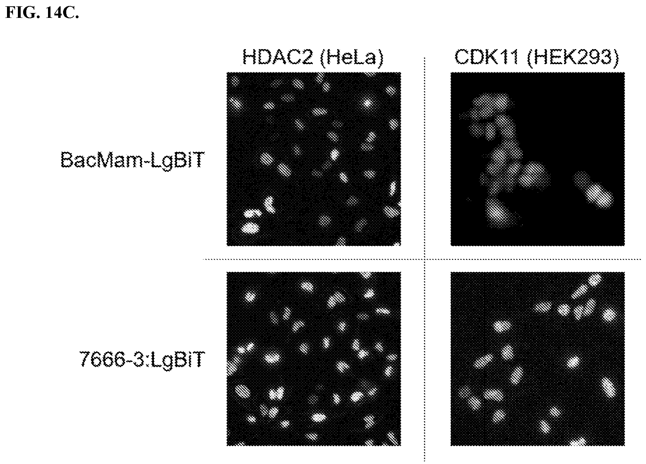

HiBiT-edited HeLa or HEK293 cells were plated at 25,000 cells/well in 400 uL in an 8-chamber slide. After 24 h, cells were transfected with PBI-7666-3•LgBiT complex. The complex was prepared by incubating 2uM LgBiT with 100 ug/mL PBI-7666-3 in Opti-MEM I reduced serum media for 30 min at room temperature. Forty microliters of the complex was added to each well. After 24 h, cells were switched to CO₂ independent media, and NanoGlo^{®} Live Cell Substrate (Promega Cat. # N2011) was added. Cells were imaged with a bioluminescence imager. Cells treated with 10% BacMam-LgBiT to transduce a CMV-LgBiT expression construct served as a control. FIG. 14A shows images of CASP3-HiBiT and EGFR-HiBiT clones of HeLa cells; FIG. 14B shows images of GSK3b-HiBiT and HDAC6-HiBit clones of HeLa cells; and FIG. 14C shows images of a HDAC2-HiBiT clone of HeLa cells and a CDK11-HiBiT clone of HEK293 cells.

### Example 4

### HaloTag-LgBiT Delivery

HeLa cells were plated at 25,000 cells per well in an 8-well chamber slide. After 24 h, cells were transfected with different complexes of PEIs (1 µg/ml) and HaloTag-LgBiT protein (200 nM). The complexes were prepared by incubating PEI (100 µg/ml) with HaloTag-LgBiT (20 µM) in Opti-MEM I reduced serum media for 30 min at room temperature. After 24 h, cells were switched to Opti-MEM I reduced serum media and then incubated with HaloTag^{®} Oregon Green^{®} Ligand (1 µM) for 30 min. Cells were washed 3 times with Opti-MEM I reduced serum media. The last wash was done by incubating the cells in the media for 30 min at 37°C under 5% (v/v) CO₂(g). Cells were counterstained with the nuclear probe NucBlue^{®} Live ReadyProbes^{®} reagent at 37°C during the final 5 min. Cells were imaged with C2 laser scanning confocal microscope (Nikon).

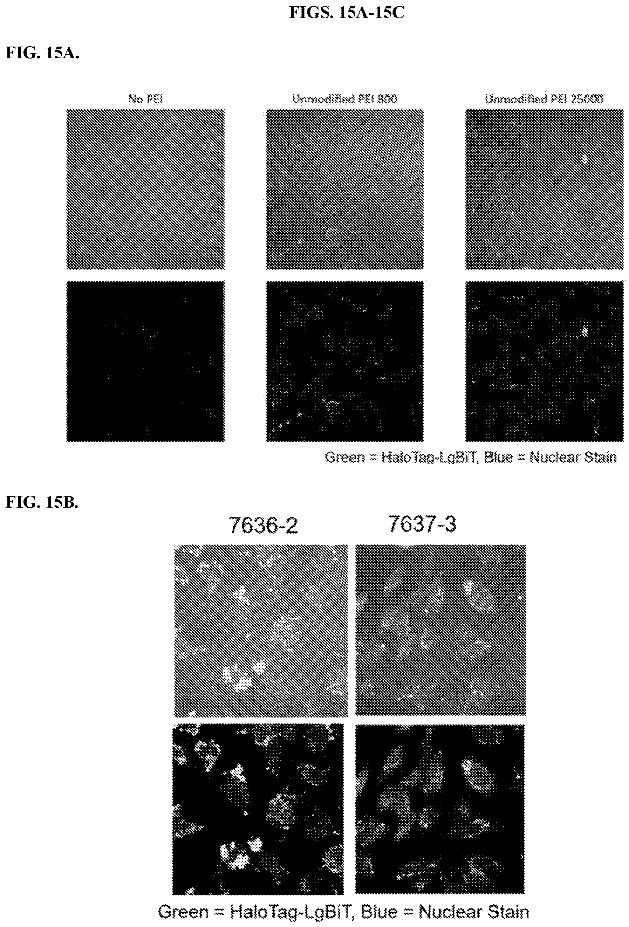

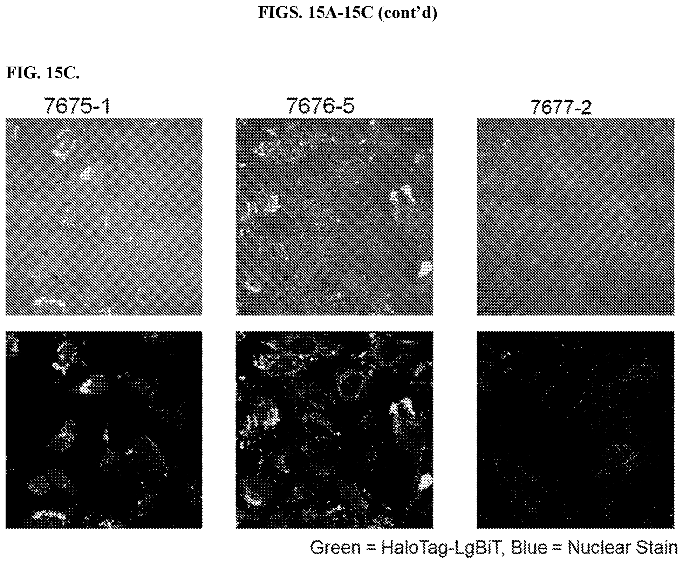

The green fluorescence intensity is proportional to the amount of intracellular HaloTag-LgBiT being delivered, which can be correlated to the efficiency of protein uptake. Modified large size PEIs (MW=25000) were better at delivering HaloTag-LgBiT than the unmodified PEI. Among the top hits, 7636-2, 7637-3, 7666-3, 7668-1, and 7676-5 worked best (FIGS. 15A-15C). Of those, three were also the best candidates for LgBiT delivery (7666-3 and 7668-1) suggesting size of the cargo influences the uptake efficiency.

Punctate staining was observed in all treatments, indicating that the majority of HaloTag-LgBiT protein resides in the endosomes. The brightness in the endosomes created a technical challenge to quantifying the portion of protein being released to the cytosol.

### Example 5

### RNP Delivery

***HiBiT Knock-in to C-terminus of GAPDH in HEK293 cells.*** Preparation of the assembly of RNP complexes and the electroporation of the complexes into cells were carried out as described previously (Schwinn et al. "CRISPR-Mediated Tagging of Endogenous Proteins with a Luminescent Peptide," ACS Chem. Biol. 2018, 13, 467-474). Briefly, in 5 µl reaction, a duplex of tracer RNA and guide RNA (to 24 µM) was prepared and subsequently was combined with 5 µl Cas9 (20 µM) to further incubate for 10 min at room temperature. Cells (2x10⁵) were resuspended in 20 µl of 4D Nucleofector solution SF for HEK293. The RNP complex (10 µl) and donor DNA (1 µl of 100 µM single-stranded oligodeoxynucleotide, ssODN) were then added to the cells. The donor DNA was designed to add the VS-HiBiT sequence to the C-terminus of the GAPDH gene. The cells were electroporated with the 4D Nucleofector System. Cells were then incubated for 5 min at room temperature and transferred to a 6-well plate for culturing.

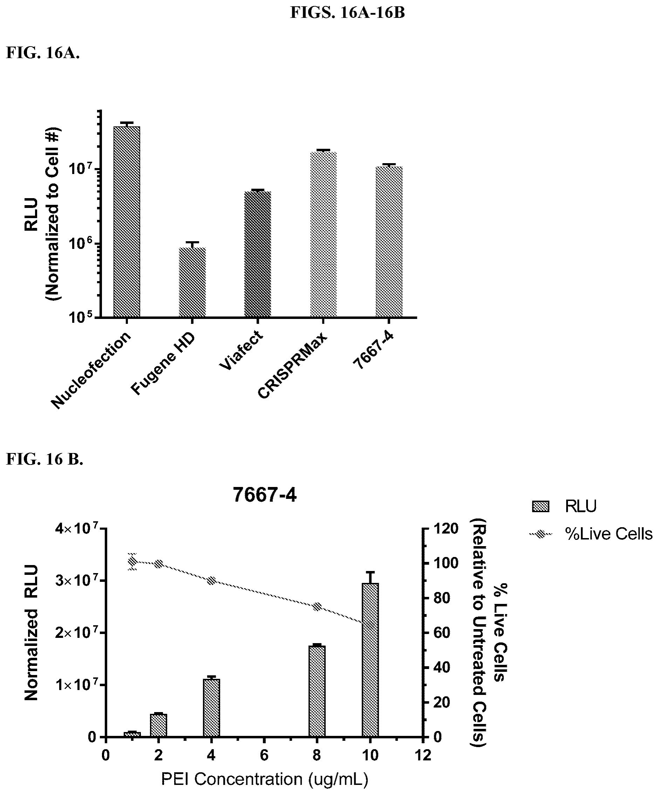

Similar procedures were used for the initial RNP delivery experiments using modified PEIs. Briefly, the RNP mixture (10 µl of 12 µM duplex guide RNA and 10 µM Cas9) was prepared as described above. After adding the donor DNA (1 µl of 100 µM) to the RNP mixture, the mixture was brought to 98 µl with Opti-MEM I reduced serum media. Modified PEI 7667-4 (2 µl of 10 mg /ml) were then added to the RNP reaction and incubated for 30 min at room temperature. The complex (100 µl) was then added to cells (2 x10⁵ in 1.9 ml of serum free media) and transferred to a 6-well plate for culturing. Cells were in serum free media for 1h. After that, cells were supplemented with FBS (10%) for growth and assays. After 24-48 h, edited cells were assayed using Nano-Glo^{®} HiBiT Lytic Reagent and CellTiter-Glo^{®} 2.0. The percentage of live cells was calculated relative to untreated cells using CellTiter-Glo^{®} Luminescent Cell Viability Assay (Promega catalog #G7570) (FIG. 16B).

The HiBiT luminescent signal normalized to the number of viable cells should be proportional to the efficiency of HiBiT insertion at the *GAPDH* locus, which may be correlated to the efficiency of RNP delivery. Nucleofection was expected to provide the most efficient RNP/ssODN delivery. As shown in FIG. 16A, Nucleofection displayed higher knock-in efficiency than FuGENE HD, ViaFect^{™}, CRISPRMax, or 7667-4 with initial conditions. However, even without optimizing the modified PEI compound or the delivery conditions, 7667-4 yielded much better RNP delivery efficiency than FuGENE HD and ViaFect^{™}, and only slightly lower than CRISPRmax.

***Model system for measuring HiBiT knock-in, target expression, and viability in the same sample**.* To screen many compounds and delivery conditions, a more informative plate-based system was needed. HiBiT was knocked in at the N-terminus of the firefly luciferase (Fluc) gene in a HEK293/CMV-Fluc stable cell line. A gRNA was designed that generated a double-stranded break within the initiating methionine codon so that repair by Non-Homologous End Joining (NHEJ) will tend to produce Insertion/Deletion (InDel) mutations that knock out expression, while Homology Directed Repair (HDR) will yield a HiBiT signal and retain the Fluc signal (Diagram 1A). CellTiter-Fluor (Promega catalog #G6080) is multiplexed for measuring cell viability so that both Fluc and HiBiT signals can be normalized to viable cells' fluorescence. The normalized ratios for Fluc signal and HiBiT signal could be used to estimate the extent of Fluc knock-out by InDel mutations and HiBiT knock-in by HDR.

When optimizing conditions for delivery of RNP/ssODN to cells using modified PEI and PAMAM polymers, a variety of concentrations of RNP, ssODN, and polymer were used. Frequently, 1.25 µM Cas9 was incubated with 1.5 µM gRNA for 10 min at room temperature to generate the RNP. Donor ssODN and modified PEI or PAMAM polymer were then added to generate an 11x solution at 250 nM RNP, 1 µM ssODN, and 22-110 µg/ml polymer. After 30 minutes incubation at room temperature, the complexes would be added to cells, diluting them 11-fold into media (e.g., 10 µl + 100 µl media in a 96-well plate). After two days, HEK293/Fluc cells were analyzed by replacing cell media with OptiMEM-I containing CellTiter-Fluor Reagent (Promega catalog #G6080) to measure the number of viable cells in the well (FIG. 17A). Fluc expression was measured using ONE-Glo EX (FIG. 17B), and HiBiT signal was measured using the HiBiT NanoDLR assay (FIG. 17C). The HiBiT signal was normalized to cell number using the HiBiT/CTF ratio (FIG. 17D). The Fluc and HiBiT signals in the well were then quantified using the Nano-Glo^{®} HiBiT Dual-Luciferase^{®} Reporter System (HiBiT NanoDLR^{™}). (Figure 17E).

FIGS. 18A-18B depict pools of HEK293/Fluc cells in which RNP/ssODN mixtures for CRISPR knock-in of HiBiT have been delivered using either nucleofection or a modified PEI or PAMAM polymer. Cell pools were expanded for multiple days prior to measurement to eliminate complications from cell death during treatment. After knock-in of HiBiT at the N-terminus of Fluc, CellTiter-Fluor and HiBiT NanoDLR were used to measure viability, Fluc expression, and HiBiT signal (FIG. 18A). The large drop in the Fluc/CTF ratio for nucleofected cells may indicate a loss of expression caused by InDel mutations. RNP/ssODN delivery mediated by the modified polymers resulted in both higher normalized Fluc expression, but also higher normalized HiBiT signal, as indicated by the HiBiT/CTF ratio. Similarly, modified PEI polymers show higher normalized HiBiT signal compared to nucleofection after delivery of an RNP/ssODN mixture designed to knock-in HiBiT at the C-terminus of GAPDH (FIG. 18B).

For other cell types, the extent of HiBiT insertion was measured using the Nano-Glo^{®} HiBiT Lytic Assay, and viability was measured using either CellTiter-Fluor in the same well or CellTiter-Glo^{®} Reagent (Promega catalog #G7570) in replicate wells.

**Diagram 1A.** A high-throughput model system for measuring HiBiT knock-in and Fluc knock-out.

Delivery of RNP/donor to
the nucleus
DSB
Firefly Luciferase
ATG
HDR
NHEJ
HiBiT
Firefly Luciferase
X
Knock-out by InDels
No cutting: High Fluc, Low HiBiT
Cutting → NHEJ: Low Fluc, Low HiBiT
Cutting → HDR: High Fluc, High HiBiT

**Diagram 1B.** gRNA and ssODN design for HiBiT knock-in in HEK293/CMV-Fluc stable cell line.

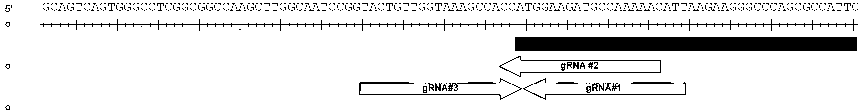
5' GCAGTCAGTGGGCCTCGGCGGCCAAGCTTGGCAATCCGGTACTGTTGGTAAAGCCACCATGGAAGATGCCAAAAACATTAAGAAGGGCCCAGCGCCATTC
gRNA #2
gRNA#3
gRNA#1

5'
GCAGTCAGTGGGCCTCGGCGGCCAAGCTTGGCAATCCGGTACTGTTGGTAAAGCCACCATGGTGAGCGGCTGGCGGCTGTTCAAGAAGATTAGCGAAGAT
900
gRNA#3
gRNA #1
gRNA#2
Donor ssODN
5'
GCCAAAAACATTAAGAAGGGCCCAGCGCCATTCTACCCACTCGAAGACGGGACCGCCGGCGAGCAGCTGCACAAAGCCATGAAGCGCTACGCCCTGGTGC
gRNA #1
gRNA#2
Donor ssODN

**Table 5. Percentage of clones with significant Fluc and HiBiT signals after single-cell sorting of pools of HEK293/Fluc cells in which HiBiT was knocked in at either the Fluc or GAPDH locus. InDel mutations generated by NHEJ-mediated repair would be expected to disrupt Fluc expression when the gRNA targets Fluc, but not GAPDH.**

| | **% High Fluc/CTF** | **% High HiBiT/CTF** | **# Colonies** |
|---|---|---|---|
| **Fluc with Nucleofection** | 12.93 | 6.61 | 234 |
| **Fluc with 7740-2** | 16.28 | 14.73 | 129 |
| **Fluc with 7740-1** | 17.44 | 18.02 | 172 |
| **Fluc with 7666-5** | 69.39 | 7.48 | 147 |
| **GAPDH with Nucleofection** | 98.88 | 2.79 | 179 |
| **GAPDH with 7741-2** | 99.51 | 6.34 | 205 |
| **GAPDH with 7759-3** | 98.96 | 7.81 | 192 |

### Example 6

### PROTAC Degradation of HiBiT-BRD4

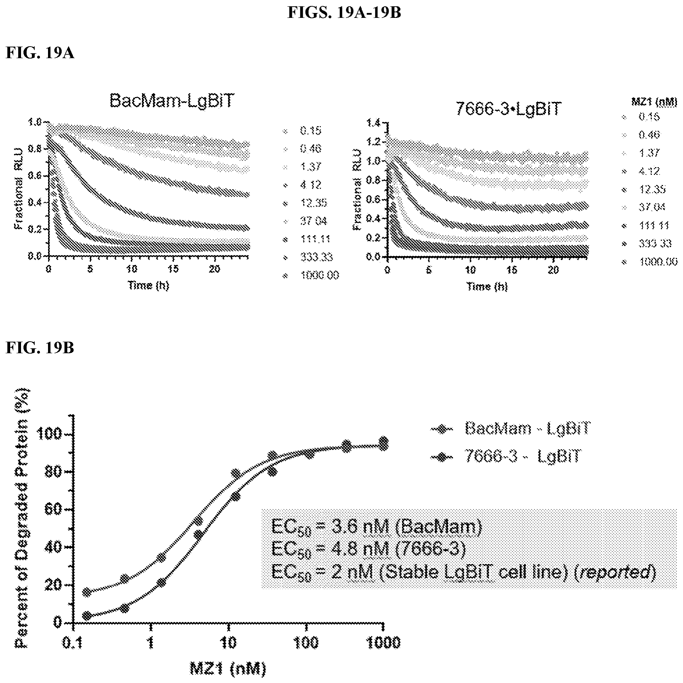

A protein degradation assay was used to demonstrate that PEI delivers functional LgBiT into cells. HiBiT-BRD4 HEK293 cells were plated at 10,000 cells/well into wells of a 96-well plate. After 24hrs, cells were transfected with a complex of PBI-7666-3 (10 ug/ml) and LgBiT protein (200nM). The complexes were prepared by incubating the PEI with LgBiT in Opti-MEM reduced serum media for 30 minutes at room temperature. Cells treated with 10% BacMam-LgBiT to transduce a CMV-LgBiT expression construct served as a control. After 21hrs, cell medium was replaced, with fresh media containing NanoGlo^{®} Endurazine Substrate (Promega Cat. # N2571) and incubated for 3 h. Next, cells were treated with the titration of MZ1, a Bromodomain BET inhibitor, to target BRD4 for degradation. Kinetic measurement was done for 24 h using a Clariostar instrument. FIG. 19A depicts the percent of degraded BRD4, which was plotted to show the similarity in response between 2 delivery methods: BacMam and PEI. FIG. 19B depicts the percent of degraded BRD4 after 24 h treatment of MZ1, and it responded in a dose-dependent manner.

### Example 7

### Removal of Extracellular LgBiT

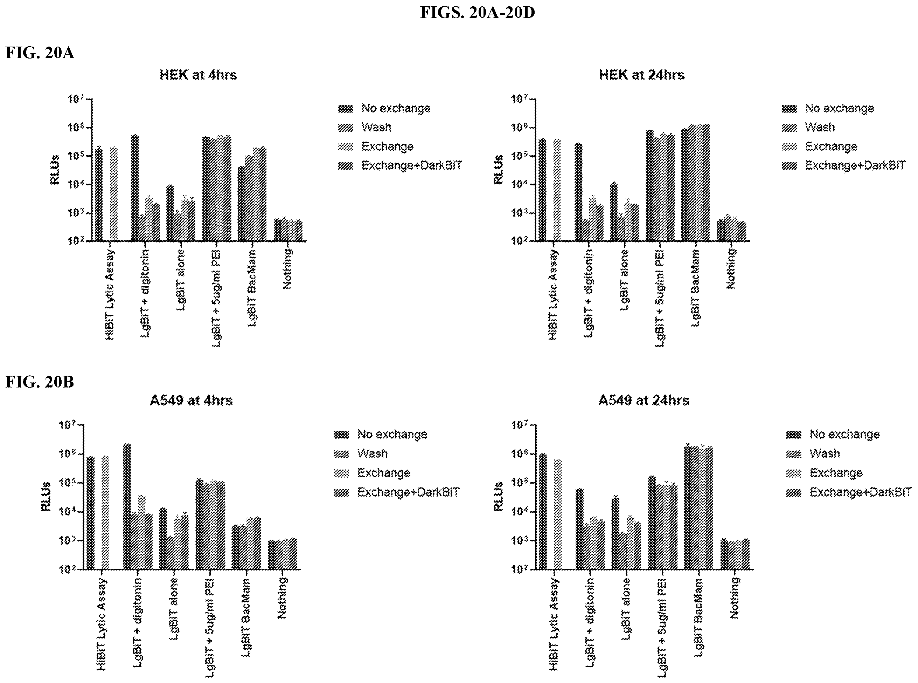

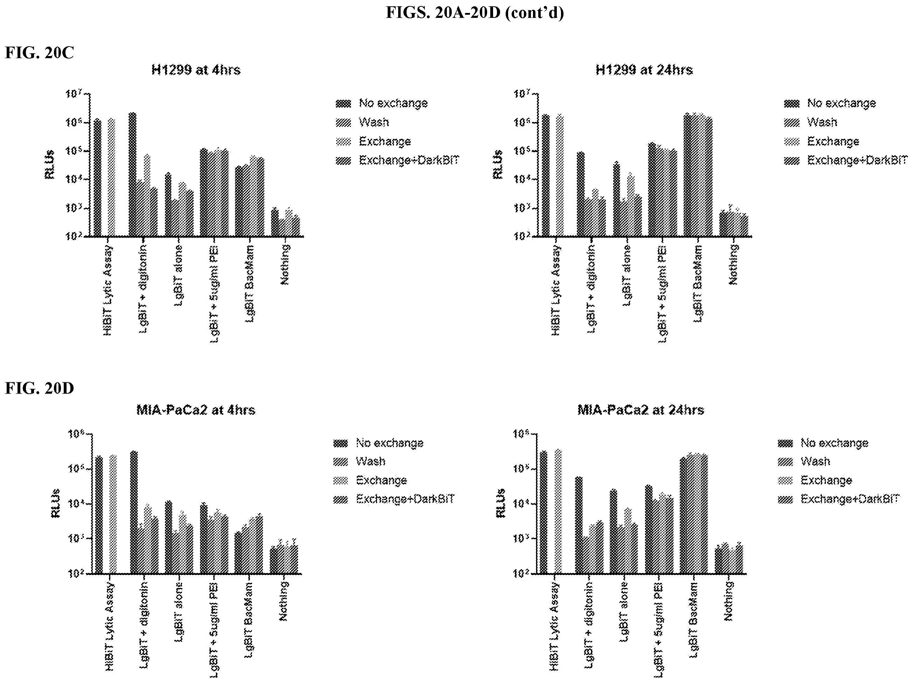

HiBiT-KRAS clones from 4 different cell backgrounds (HEK293, A549, H1299, or MiaPaCa-2) were plated at 10,000 cells/well into wells of a 96-well plate. After 24hrs, cells were transfected with a complex of PBI-7666-3 (5ug/ml) and LgBiT protein (200 nM). The complexes were prepared by incubating the PEI with LgBiT in Opti-MEM reduced serum media for 30 minutes at room temperature. Cells treated with 10% BacMam-LgBiT to transduce a CMV-LgBiT expression construct served as a control. Cells were treated with digitonin (50 ug/mL) and LgBiT protein (200 nM). Four conditions were examined to differentiate intracellular versus extracellular luminescence signals. The "No Exchange Condition" is when cells were treated with LgBiT•7666-3 for either 4 h or 24 h, and luminescence is measured at the given time without media exchange. "Exchange Condition" is when cells, post LgBiT delivery, were exchanged for fresh media in either absence or presence of DarkBiT (SEQ ID NO: 6) (1 uM). DarkBiT peptide, a cell impermeable peptide, is needed to deactivate extracellular LgBiT. "Wash Condition" is when cells, post LgBiT delivery, were washed one time with fresh media, replaced with fresh media, and followed by luminescence measurement. The wash condition reflects the intracellular measurement of NanoBiT. Exchange conditions removed some, but not all extracellular LgBiT; thus luminescence signal was lower than with the no exchange condition, but higher than wash condition. At the given time, cells were assayed using NanoGlo^{®} Live Cell Assay System (Promega Cat. # N2011) and CellTiter-Glo^{®} 2.0 Assay (Promega Cat. No. G7570). Luminescence was measured on a GloMax^{®} Discover. Results are shown in FIGS. 20A-20D.

The PEI-based system delivers functional LgBiT into cells. The intracellular signal obtained from the PEI method was similar between 4 h and 24 h incubation time, suggesting the method requires a minimum of 4 h incubation time to reach its saturation signal. In contrast, BacMam transduction delivers nucleic acid. For each of the cell lines tested, the signal derived from BacMam was much lower than that of the PEI method after 4 h incubation time. After 24 h incubation, the signal was comparable between the PEI method and BacMam transduction in the case of HEK293 cells. Of the other three cases, BacMam transduction was better at delivering LgBiT than the modified PEI compound.

It is understood that the foregoing detailed description and accompanying examples are merely illustrative and are not to be taken as limitations upon the scope of the disclosure, which is defined solely by the appended claims and their equivalents.

Various changes and modifications to the disclosed embodiments will be apparent to those skilled in the art. Such changes and modifications, including without limitation those relating to the chemical structures, substituents, derivatives, intermediates, syntheses, compositions, formulations, or methods of use of the disclosure, may be made without departing from the spirit and scope thereof.

The sequences provide below are referenced herein and are provided as part of an accompanying sequence listing and sequence listing statement:
***WT OgLuc (SEQ ID NO: 1)*** 
MFTLADFVGDWQQTAGYNQDQVLEQGGLSSLFQALGVSVTPIQKVVLSGEN
GLKADIHVIIPYEGLSGFQMGLIEMIFKVVYPVDDHHFKIILHYGTLVIDGVTPNMIDYFG
RPYPGIAVFDGKQITVTGTLWNGNKIYDERLINPDGSLLFRVTINGVTGWRLCENILA
***NanoLuc (SEQ ID NO: 2)*** 
MVFTLEDFVGDWRQTAGYNLDQVLEQGGVSSLFQNLGVSVTPIQRIVLSGEN
GLKIDIHVIIPYEGLSGDQMGQIEKIFKVVYPVDDHHFKVILHYGTLVIDGVTPNMIDYFG
RPYEGIAVFDGKKITVTGTLWNGNKIIDERLINPDGSLLFRVTINGVTGWRLCERILA
***HiBiT (SEQ ID NO: 3)***
   VSGWRLFKKIS
***LgBiT (SEQ ID NO: 4)*** 
MVFTLEDFVGDWRQTAGYNLDQVLEQGGVSSLFQNLGVSVTPIQRIVLSGEN
GLKIDIHVIIPYEGLSGDQMGQIEKIFKVVYPVDDHHFKVILHYGTLVIDGVTPNMIDYFG
RPYEGIAVFDGKKITVTGTLWNGNKIIDERLINPDGSLLFRVTIN
***SmBiT (SEQ ID NO: 5)***
   VTGYRLFEEIL
***DarkBiT (SEQ ID NO: 6)***
   VSGWALFKKIS
***DarkBiT (SEQ ID NO: 7)***
   MVSGWALFKKIS
***DarkBiT (SEQ ID NO: 8)***
   MGVTGWRLCERILA
***LgTrip 3092 (SEQ ID NO: 9)*** 
VFTLDDFVGDWEQTAAYNLDQVLEQGGVSSLLQNLAVSVTPIMRIVRSGENA
LKIDIHVIIPYEGLSADQMAQIEEVFKVVYPVDDHHFKVILPYGTLVIDGVTPNKLNYFGR
PYEGIAVFDGKKITVTGTLWNGNKIIDERLITPD
***LgTrip 3546 (SEQ ID NO: 10)*** 
VFTLDDFVGDWEQTAAYNLDQVLEQGGVSSLLQNLAVSVTPIMRIVRSGENA
LKIDIHVIIPYEGLSADQMAQIEEVFKVVYPVDDHHFKVILPYGTLVIDGVTPNKLNYFGR
PYEGIAVFDGKKITTTGTLWNGNKIIDERLITPD
***LgTrip 2098 (SEQ ID NO: 11)*** 
VFTLDDFVGDWEQTAAYNLDQVLEQGGVSSLLQNLAVSVTPIMRIVRSGENA
LKIDIHVIIPYEGLSADQMAQIEEVFKVVYPVDDHHFKVILPYGTLVIDGVTPNKLNYFGR
PYEGIAVFDGKKITTTGTLWNGNKIIDERLITPD
***SmTrip9 (SEQ ID NO: 12)***
   GSMLFRVTINS
The following paragraphs represent possible embodiments according to the invention:
1. A compound or a salt thereof, the compound comprising:
   a polyethyleneimine polymer; and
   a plurality of substituents bound to amino groups of the polyethyleneimine polymer,
   wherein each substituent independently has a formula (I):

   -X-(CH₂)ₙ-Z (I),

   wherein:
   X is a bond or -C(O)-O-;
   n is 0, 1, 2, 3, 4, or 5; and
   Z is selected from a haloalkyl group, an aryl group, a substituted aryl group, a heteroaryl group, and a substituted heteroaryl group.
2. The compound of paragraph 1, or a salt thereof, wherein the polyethyleneimine polymer has a weight average molecular weight of about 500 Da to about 250000 Da.
3. The compound of paragraph 2, or a salt thereof, wherein the polyethyleneimine polymer has a weight average molecular weight of about 500 Da to about 2000 Da.
4. The compound of paragraph 2, or a salt thereof, wherein the polyethyleneimine polymer has a weight average molecular weight of about 5000 Da to about 250000 Da.
5. The compound of any one of paragraphs 1-4, or a salt thereof, wherein the polyethyleneimine polymer is a branched polyethyleneimine polymer.
6. The compound of any one of paragraphs 1-4, or a salt thereof, wherein the polyethyleneimine polymer is a linear polyethyleneimine polymer.
7. The compound of any one of paragraphs 1-6, or a salt thereof, wherein Z is a haloalkyl group having the following formula:

   -(CF₂)ₘ-CF₃,

   wherein m is 1, 2, 3, 4, 5, 6, 7, 8, 9, or 10.
8. The compound of any one of paragraphs 1-6, or a salt thereof, wherein Z is a pentafluorophenyl group.
9. The compound of any one of paragraphs 1-6, or a salt thereof, wherein Z is an unsubstituted pyridyl group.
10. The compound of any one of paragraphs 1-9, or a salt thereof, wherein:
   X is -C(O)O-; and
   n is 1 or 2.
11. The compound of paragraph 10, or a salt thereof, wherein Z is a haloalkyl group having the following formula:

   -(CF₂)ₘ-CF₃,

   wherein m is 1, 2, 3, 4, or 5.
12. The compound of any one of paragraphs 1-9, or a salt thereof, wherein
   X is a bond; and
   n is 1 or 2.
13. The compound of any one of paragraphs 1-12, or a salt thereof, wherein about 0.1 mol% to about 60 mol% of the amino groups of the polyethyleneimine polymer are bound to a substituent of formula (I).
14. The compound of paragraph 13, or a salt thereof, wherein about 5 mol% to about 50 mol% of the amino groups of the polyethyleneimine polymer are bound to a substituent of formula (I).
15. The compound of paragraph 14, or a salt thereof, wherein about 8 mol% to about 40 mol% of the amino groups of the polyethyleneimine polymer are bound to a substituent of formula (I).
16. A compound or a salt thereof, the compound comprising:
   a poly(amidoamine) dendrimer; and
   a plurality of substituents bound to amino groups of the poly(amidoamine) dendrimer,
   wherein each substituent independently has a formula (I):

   -X-(CH₂)ₙ-Z (I),

   wherein:
   X is a bond or -C(O)-O-;
   n is 0, 1 or 2; and
   Z is selected from a haloalkyl group, an aryl group, a substituted aryl, a heteroaryl group, and a substituted heteroaryl group.
17. The compound of paragraph 16, or a salt thereof, wherein the poly(amidoamine) dendrimer is a Generation 1, Generation 2, Generation 3, Generation 4, Generation 5, Generation 6, Generation 7, Generation 8, Generation 9, or Generation 10 poly(amidoamine) dendrimer.
18. The compound of paragraph 17, or a salt thereof, wherein the poly(amidoamine) dendrimer is a Generation 1, Generation 2, Generation 3, or Generation 4 poly(amidoamine) dendrimer.
19. The compound of any one of paragraphs 16-18, or a salt thereof, wherein Z is a haloalkyl group having the following formula:

   -(CF₂)ₘ-CF₃,

   wherein m is 1, 2, 3, 4, 5, 6, 7, 8, 9, or 10.
20. The compound of any one of paragraph 16-19, or a salt thereof, wherein Z is a pentafluorophenyl group.
21. The compound of any one of paragraphs 16-19, or a salt thereof, wherein Z is an unsubstituted pyridyl group.
22. The compound of any one of paragraphs 16-21, or a salt thereof, wherein:
   X is -C(O)O-.
23. The compound in paragraph 22, or a salt thereof, wherein Z is a haloalkyl group having the following formula:

   -(CF₂)ₘ-CF₃,

   wherein m is 1, 2, 3, 4, or 5.
24. The compound of any one of paragraphs 16-23, or a salt thereof, wherein:
   X is a bond; and
   n is 1.
25. The compound of any one of paragraphs 16-24, or a salt thereof, wherein about 0.1 mol% to about 80 mol% of the primary amino groups of the poly(amidoamine) dendrimer are bound to a substituent of formula (I).
26. The compound of paragraph 25, wherein about 10 mol% to about 70 mol% of the primary amino groups of the poly(amidoamine) dendrimer are bound to a substituent of formula (I).
27. The compound of paragraph 26, wherein about 20 mol% to about 70 mol% of the primary amino groups of the poly(amidoamine) dendrimer are bound to a substituent of formula (I).
28. A method of delivering a biomolecule to a cell, comprising:
   contacting the cell with an effective amount of a compound of any one of paragraphs 1-27, or a salt thereof; and
   contacting the cell with the biomolecule.
29. The method of paragraph 28, wherein the method comprises contacting the cells with an effective amount of two or more different compounds of any one of paragraphs 1-27, or salts thereof.
30. The method of paragraph 28 or 29, wherein the biomolecule is at least one of a deoxyribonucleic acid (DNA) molecule, a ribonucleic acid (RNA) molecule, a peptide, a polypeptide, a protein, or any combinations or derivatives thereof.
31. The method of paragraph 28 or 29, wherein the biomolecule is a deoxyribonucleic acid (DNA) molecule or a ribonucleic acid (RNA) molecule.
32. The method of paragraph 28 or 29, wherein the biomolecule is a peptide or polypeptide capable of luminescent activity.
33. The method of paragraph 28 or paragraph 29, wherein the biomolecule comprises a polypeptide sequence of SEQ ID NO: 4.
34. The method of paragraph 28 or 29, wherein the biomolecule is a ribonucleoprotein complex comprising a Cas9 protein.
35. The method of paragraph 34, wherein the ribonucleoprotein complex further comprises a guide RNA (gRNA) and a donor DNA template, wherein the donor DNA template comprises a sequence encoding a polypeptide from SEQ ID NO: 3.
36. The method of any one of paragraphs 28-35, comprising mixing the compound and the biomolecule to form a mixture, and subsequently contacting the cell with the mixture.
37. A kit comprising a compound of any one of paragraphs 1-27, or a salt thereof.
38. The kit of paragraph 37, wherein the kit comprises the compound or the salt thereof in a container.
39. The kit of paragraph 37 or 38, further comprising at least one of a DNA molecule, an RNA molecule, a peptide, a polypeptide, a protein, or any combinations or derivatives thereof.
40. The kit of paragraph 39, wherein the peptide comprises a Cas9 protein.
41. The kit of paragraph 39, wherein the DNA molecule is a donor DNA template comprising a sequence encoding a polypeptide from SEQ ID NO: 3.
42. The kit of any one of paragraphs 37-41, further comprising instructions for using the compound or the salt thereof for transfection of a biomolecule.
43. A method for altering a sequence of an endogenous protein in a cell, the method comprising:
   assembling a ribonucleoprotein (RNP) complex comprising a Cas9 protein, a donor DNA template, and a guide RNA; and
   delivering the RNP complex into a cell using a compound of any of paragraphs 1-27.
44. A method for tagging an endogenous protein in a cell, the method comprising:
   assembling a ribonucleoprotein (RNP) complex comprising a Cas9 protein, a donor DNA template, and a guide RNA, wherein the donor DNA template comprises a sequence encoding a peptide or polypeptide tag sequence; and
   delivering the RNP complex into a cell using a compound of any of paragraphs 1-27.
45. The method of paragraph 44, wherein the donor DNA template comprises a sequence encoding a peptide tag selected from SEQ ID NO: 3 and SEQ ID NO: 5.
46. The method of paragraph 44, wherein the donor DNA template further comprises homology arms flanking the sequence encoding peptide or polypeptide tag sequence.

## Claims

1. A compound or a salt thereof, the compound comprising:
a Generation 1, Generation 2, Generation 3, or Generation 4 poly(amidoamine) dendrimer; and
a plurality of substituents bound to amino groups of the poly(amidoamine) dendrimer,
wherein each substituent independently has a formula (I):
-X-(CH₂)ₙ-Z (I),
wherein:
X is -C(O)-O-;
n is 1 or 2; and
Z is a haloalkyl group having the formula -(CF₂)ₘ-CF₃, wherein m is 1, 2, 3, 4, 5, 6, 7, 8, 9, or 10.

2. The compound in claim 1, or a salt thereof, wherein Z is a haloalkyl group having the following formula:
-(CF₂)ₘ-CF₃,
wherein m is 1, 2, 3, 4, or 5.

3. The compound of claim 1 or claim 2, or a salt thereof, wherein about 0.1 mol% to about 80 mol% of the primary amino groups of the poly(amidoamine) dendrimer are bound to a substituent of formula (I); optionally wherein about 10 mol% to about 70 mol% of the primary amino groups of the poly(amidoamine) dendrimer are bound to a substituent of formula (I); optionally wherein about 20 mol% to about 70 mol% of the primary amino groups of the poly(amidoamine) dendrimer are bound to a substituent of formula (I).

4. A method of delivering a biomolecule to a cell, comprising:
contacting the cell with an effective amount of a compound of any one of claims 1-3, or a salt thereof; and
contacting the cell with the biomolecule; optionally wherein the method comprises contacting the cells with an effective amount of two or more different compounds of any one of claims 1-3, or salts thereof.

5. The method of claim 4, wherein the biomolecule is at least one of a deoxyribonucleic acid (DNA) molecule, a ribonucleic acid (RNA) molecule, a peptide, a polypeptide, a protein, or any combinations or derivatives thereof.

6. The method of claim 4, wherein the biomolecule is a peptide or polypeptide capable of luminescent activity; optionally wherein the biomolecule comprises a polypeptide sequence of SEQ ID NO: 4.

7. The method of claim 4, wherein the biomolecule is a ribonucleoprotein complex comprising a Cas9 protein; optionally wherein the ribonucleoprotein complex further comprises a guide RNA (gRNA) and a donor DNA template, wherein the donor DNA template comprises a sequence encoding a polypeptide from SEQ ID NO: 3.

8. The method of any one of claims 4-7, comprising mixing the compound and the biomolecule to form a mixture, and subsequently contacting the cell with the mixture.

9. A kit comprising a compound of any one of claims 1-3, or a salt thereof; optionally wherein the kit comprises the compound or the salt thereof in a container.

10. The kit of claim 9, further comprising at least one of a DNA molecule, an RNA molecule, a peptide, a polypeptide, a protein, or any combinations or derivatives thereof.

11. The kit of claim 10, wherein the peptide comprises a Cas9 protein or wherein the DNA molecule is a donor DNA template comprising a sequence encoding a polypeptide from SEQ ID NO: 3.

12. The kit of any one of claims 9-11, further comprising instructions for using the compound or the salt thereof for transfection of a biomolecule.

13. A method for altering a sequence of an endogenous protein in a cell, the method comprising:
assembling a ribonucleoprotein (RNP) complex comprising a Cas9 protein, a donor DNA template, and a guide RNA; and
delivering the RNP complex into a cell using a compound of any of claims 1-3.

14. A method for tagging an endogenous protein in a cell, the method comprising:
assembling a ribonucleoprotein (RNP) complex comprising a Cas9 protein, a donor DNA template, and a guide RNA, wherein the donor DNA template comprises a sequence encoding a peptide or polypeptide tag sequence; and
delivering the RNP complex into a cell using a compound of any of claims 1-3.

15. The method of claim 14, wherein the donor DNA template comprises a sequence encoding a peptide tag selected from SEQ ID NO: 3 and SEQ ID NO: 5 or wherein the donor DNA template further comprises homology arms flanking the sequence encoding peptide or polypeptide tag sequence.
